(19) Europäisches Patentamt / European Patent Office / Office européen des brevets

(11) **EP 4 000 657 A1**

(12) **EUROPEAN PATENT APPLICATION**

(43) Date of publication:
**25.05.2022 Bulletin 2022/21**

(21) Application number: **20383002.1**

(22) Date of filing: **18.11.2020**

(51) International Patent Classification (IPC):
**A61L 15/00** (2006.01)   **B01J 27/18** (2006.01)
**C01B 25/32** (2006.01)   **B01D 53/00** (2006.01)
**C07C 227/00** (2006.01)

(52) Cooperative Patent Classification (CPC):
(C-Sets available)
**B01J 35/002; B01D 53/86; B01J 27/18;**
**B01J 37/08; B01J 37/342; C07C 227/12;**
B01D 2255/2045; B01D 2257/502; B01D 2257/504;
B01D 2257/7025; B01D 2258/06      (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **B. Braun Surgical, S.A.**
  **08191 Rubi (Barcelona) (ES)**
• **Universitat Politècnica De Catalunya**
  **08034 Barcelona (ES)**

(72) Inventors:
• **ALEMAN LLANSO, Carlos Enrique**
  **08019 Barcelona (ES)**

• **PUIGGALI BELLALTA, Jordi**
  **08019 Barcelona (ES)**
• **SANS, Jordi**
  **08019 Barcelona (ES)**
• **TURON DOLS, Pau**
  **08191 Rubi (Barcelona) (ES)**
• **SANZ BELTRAN, Vanesa**
  **08191 Rubi (Barcelona) (ES)**
• **RODRIGUEZ RIVERO, Anna Maria**
  **08191 Rubi (Barcelona) (ES)**

(74) Representative: **Patentanwälte**
**Ruff, Wilhelm, Beier, Dauster & Partner mbB**
**Kronenstraße 30**
**70174 Stuttgart (DE)**

(54) **PROCESS FOR PRODUCING AMINO ACIDS AND A USE THEREOF**

(57)    The invention relates to a process for producing amino acids, in particular glycine and/or alanine, comprising the step of
- contacting a gas mixture comprising or consisting of carbon dioxide, methane and nitrogen in presence of water with a catalyst comprising or consisting of permanently polarized hydroxyapatite and brushite and/or a brushite-like material.

   Further the invention relates to the use of the process for removing harmful gases from an atmosphere.

(a)

EP 4 000 657 A1

**(Cont. next page)**

(52) Cooperative Patent Classification (CPC): (Cont.)

C-Sets
**C07C 227/12**

**Description**

FIELD OF THE INVENTION

The present invention relates to a process of producing amino acids, in particular glycine and/or alanine, and a use thereof.

BACKGROUND OF THE INVENTION

Synthetic mineral hydroxyapatite (HAp), a crystalline form of calcium phosphate with formula $Ca_{10}(PO_4)_6(OH)_2$, is of major biomedical interest because of its compositional and structural similarity with bones and teeth, which has motivated its use to repair and reconstruct such hard tissues. HAp has a hexagonal structure with space group $P6_3/m$ and cell dimensions a=b= 9.42 Å and c= 6.87 Å, which is stable up to 1273 °C.

The electrical properties of HAp were found to influence its biomedical application. Thus, there was an early interest in the polarization of HAp to generate a surface charge by applying a DC electric potential (*i.e.* from 1.0 to 10.0 kV/cm) at elevated temperatures, i.e. from 200 °C to 800 °C (Itoh, S.; Nakamura, S.; Kobayashi, T.; Shinomiya, K.; Yamashita, K.; Itoh, S. Effect of Electrical Polarization of Hydroxyapatite Ceramics on New Bone Formation. Calcif. Tissue Int. 2006, 78, 133-142).

Such thermally stimulated polarization (TSP) process causes defects inside crystal grains and originates space charge polarization in the grain boundaries, both inducing the formation of electrical dipoles (Nakamura, S.; Kobayashi, T.; Yamashita, K. Highly Oriented Calcification in Newly Formed Bones on Negatively Charged Hydroxyapatite Electrets. Key Eng. Mater. 2005, 284-286, 897-900).

However, the relaxation of such dipoles through time suggested that polarization was only partially maintained (semi-permanently), even though this effect was not quantified.

Recently, permanently polarized hydroxyapatite was synthesized by applying a constant DC voltage of 500 V (*i.e.* DC field of 3 kV/cm) at 1000 °C for 1 h to previously sintered crystalline HAp (cHAp) Rivas, M.; del Valle, L. J.; Armelin, E.; Bertran, O.; Turon, P.; Puiggalí, J.; Alemán, C. Hydroxyapatite with Permanent Electrical Polarization: Preparation, Characterization, and Response against Inorganic Adsorbates. Chem. Phys. Chem. 2018, 19, 1746-1755). This TSP process caused important chemical changes, as the formation of OH⁻ defects (vacancies) and structural variations that resulted in an increment of the crystallinity. Consequently, the electrochemical properties and electrical conductivity of the resulting polarized mineral increased noticeably when compared with cHAp (*i.e.* sintered HAp without TSP treatment).

For example, it was found that permanently polarized hydroxyapatite may be used as an electrophotocatalyst to obtain both glycine (Gly) and alanine (Ala; D/L racemic mixture) in mild reaction conditions (i.e. from atmospheric pressure to 6 bars and 95 °C) by fixing nitrogen from $N_2$ and carbon from $CO_2$ and $CH_4$ (Rivas, M.; del Valle, L. J.; Turon, P.; Alemán, C.; Puiggalí, J. Sustainable Synthesis of Amino Acids by Catalytic Fixation of Molecular Dinitrogen and Carbon Dioxide. Green Chem. 2018, 20, 685-693).

Despite the progress already made, there remains further need for a process, in particular an improved process, for producing amino acids.

OBJECT AND SOLUTION

In view of the foregoing, the object underlying the present invention is therefore to make available a process for producing amino acids, in particular glycine and/or alanine, which addresses the afore-mentioned need.

This object is accomplished by a process according to independent claim 1 and by the use according to claim 16. Preferred embodiments of the process are defined in the dependent claims 2 to 15. Further preferred embodiments of the invention are defined in the present description. The subject-matter and wording, respectively of all claims is hereby incorporated into the description by explicit reference.

The present invention relates to a process for producing or synthesizing amino acids, in particular natural amino acids, preferably glycine and/or alanine.

The process comprises the step of

- contacting a gas mixture comprising or consisting of carbon dioxide ($CO_2$), methane ($CH_4$) and nitrogen ($N_2$) in presence of water ($H_2O$) with a catalyst, wherein the catalyst comprises or consists of permanently polarized hydroxyapatite and brushite or wherein the catalyst comprises or consists of permanently polarized hydroxyapatite and a brushite-like material or wherein the catalyst comprises or consists of permanently polarized hydroxyapatite, brushite and a brushite-like material.

Hereinafter, the above step of the inventive process is denoted as "contacting step".

Further, hereinafter and if not specified otherwise, the term "catalyst" means the above catalyst, i.e. a catalyst comprising or consisting of permanently polarized hydroxyapatite and brushite or a catalyst comprising or consisting of permanently polarized hydroxyapatite and a brushite-like material or a catalyst comprising or consisting of permanently polarized hydroxyapatite, brushite and a brushite-like material.

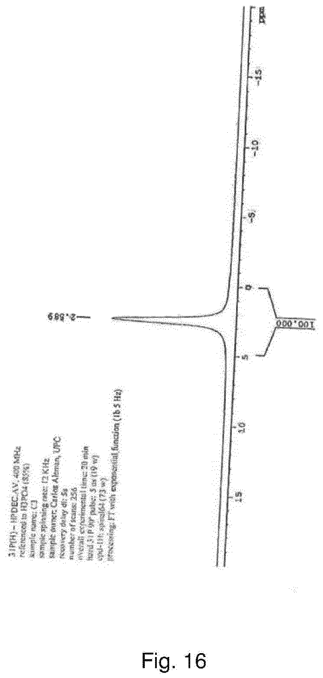

The term "permanently polarized hydroxyapatite" as used according to the present invention means a hydroxyapatite, in particular a synthetic hydroxyapatite, that has undergone a complete structural redistribution, in particular almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over. The corresponding $^{31}$P-NMR spectrum of the permanently polarized hydroxyapatite is as shown on fig. 16.

The term "thermally polarized hydroxyapatite" as used according to the present invention preferably means a permanently polarized hydroxyapatite obtained or obtainable by a process (thermal polarization process) comprising the steps of

(a) sintering a sample of hydroxyapatite and/or amorphous calcium phosphate, in particular at a temperature between 700 °C and 1200 °C, and

(b) applying a constant or variable DC voltage between 250 V and 2500 V, in particular for at least 1 min and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or

applying an equivalent field between 1.49 kV/cm and 15 kV/cm, in particular for at least 1 min and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or
applying an electrostatic discharge between 2500 V and 1500000 V, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a) or
applying an equivalent electrical field between 148.9 kV/cm and 8928 kV/cm, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (a) or to a shaped body obtained from the sintered sample of hydroxyapatite and/or amorphous calcium phosphate obtained in step (a).

The sample of hydroxyapatite in step (a) may be a sample of a natural, i.e. naturally occurring, hydroxyapatite or of a synthetic hydroxyapatite.

Further, the sample of hydroxyapatite in step (a) may be in particular a sample of crystalline hydroxyapatite.

Accordingly, the permanently polarized hydroxyapatite of the composition or material according to the present invention is preferably obtained or obtainable by the above process (thermal polarization process).

The term "brushite" as used according to the present invention means a phosphate material or phosphate mineral, in particular synthetic phosphate material or synthetic phosphate mineral, with the chemical formula $CaHPO_4 \cdot 2H_2O$. In the WAXS (wide angle x-ray scattering) spectrum, the most representative peaks of brushite appear at $2\theta = 29°$, $31°$, $35°$, $42°$, and $51°$, which have been attributed to the $(141)$, $(22\bar{1})$ $(121)$, $(15\bar{2})$ and $(14\bar{3})$ reflections, respectively (JCPDS card number 72-0713).

The term "brushite-like material" as used according to the present invention refers to a calcium phosphate material that presents in the Raman spectrum peaks at 878 cm$^{-1}$, 848 cm$^{-1}$ and 794 cm$^{-1}$, which correspond to the normal vibration mode of $HPO_4^{2-}$, the POH deformation mode and the POH rotation mode, respectively.

The term "room temperature" as used according to the present invention means a temperature from 15 °C to 35 °C, in particular 18 °C to 30 °C, preferably 20 °C to 30 °C, more preferably 20 °C to 28 °C, particularly 20 °C to 25 °C.

The present invention rests on the surprising finding that apparition or formation of brushite or a brushite-like material on permanently polarized hydroxyapatite results in a new and versatile catalyst. Further, it surprisingly turned out that said catalyst can be successfully used in a reaction for producing or synthesizing amino acids, in particular glycine and/or alanine, in particular under mild conditions (particularly < 10 bar pressure and ≤ 250 °C, in particular < 250 °C, temperature) and with lower levels of environmental contamination and cost. The catalyst can be in particular obtained by applying a thermally stimulated polarization (TSP) treatment to hydroxyapatite, in particular sintered hydroxyapatite, wherein the hydroxyapatite is spaced from a positive electrode which together with a negative electrode is used for carrying out the TSP treatment.

Preferably, the catalyst according to the present invention is an electrocatalyst, in particular a photoelectrocatalyst.

In an embodiment of the invention, the catalyst is in the form of a multi-phase, in particular biphase, catalyst, wherein the permanently polarized hydroxyapatite forms a phase, in particular a main phase, of the catalyst and the brushite and/or the brushite-like material form/forms a further phase of the catalyst. Preferably, the brushite and/or the brushite-like material is formed or present on a surface of the permanently polarized hydroxyapatite. In particular, the permanently polarized hydroxyapatite, to be more precise a surface thereof, is at least partially, in particular only partially or completely, layered or covered by the brushite and/or the brushite-like material.

The term "main phase" as used according to the present invention in the context of the catalyst means a phase having a proportion, related to the total weight of the catalyst, which is larger than a proportion, also related to the total weight of the catalyst, of remaining phases or a remaining phase of the catalyst.

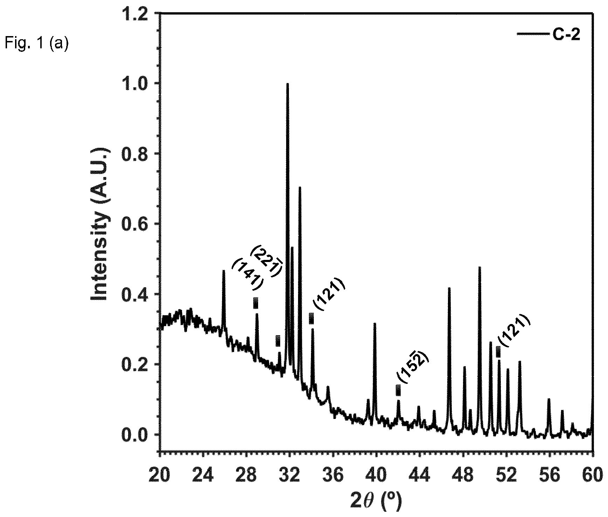

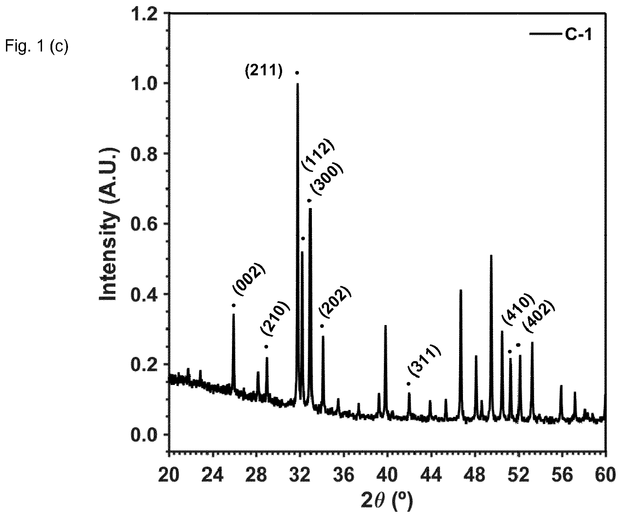

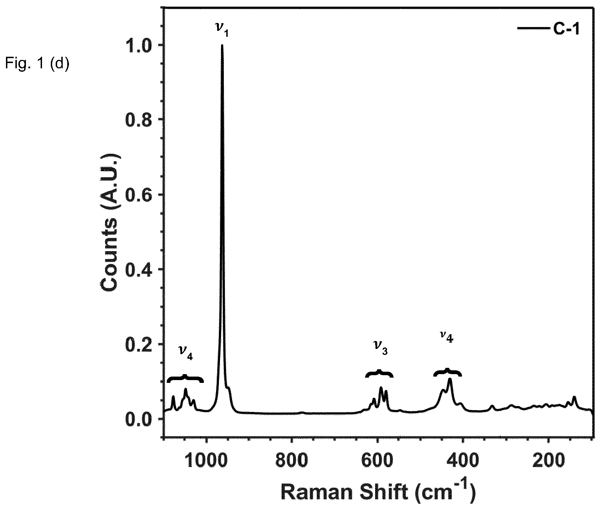

In a further embodiment, the catalyst has a wide angle x-ray scattering (WAXS) pattern as shown on fig. 1(a). The x-ray scattering (WAXS) pattern shows peaks, in particular representative or unique peaks, at $2\theta = 29°$, $31°$, $35°$, $42°$, and $51°$, which have been attributed to the $(141)$, $(22\bar{1})$ $(121)$, $(15\bar{2})$ and $(14\bar{3})$ reflections, respectively (JCPDS card number 72-0713). Preferably, said pattern is carried out or obtained at a room temperature, preferably at a temperature of 20 °C to 25 °C and/or under atmospheric conditions, in particular under atmospheric humidity and/or atmospheric pressure.

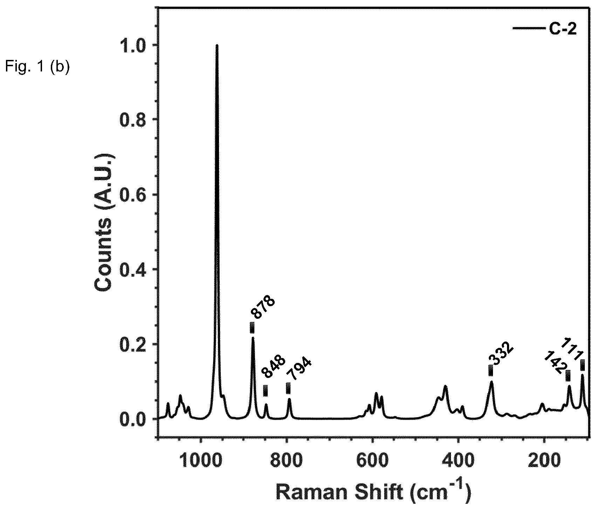

In a further embodiment, the catalyst has a Raman spectrum as shown on fig. 1(b). The Raman spectrum shows peaks at 878 cm$^{-1}$, 848 cm$^{-1}$ and 794 cm$^{-1}$. Said peaks correspond to the normal vibration mode of $HPO4^{2-}$, the POH deformation mode and the POH rotation mode, respectively. Preferably, said spectrum is carried out or obtained at a room temperature, preferably at a temperature of 20 °C to 25 °C and/or under atmospheric conditions, in particular under atmospheric humidity and/or atmospheric pressure, in particular recorded using a laser at 532 nm.

Further, the permanently polarized hydroxyapatite has preferably a $^{31}$P-NMR spectrum as shown on figure 16. Said spectrum shows a unique peak at 2.6 ppm or around 2.6 ppm, i.e. in the range of 2.5 ppm to 2.7 ppm, corresponding to phosphate groups of hydroxyapatite. Preferably, said spectrum is carried out or obtained with solid permanently polarized hydroxyapatite at a temperature of 20 °C to 25 °C and using phosphoric acid ($H_3PO_4$) as a reference.

Further, the permanently polarized hydroxyapatite may have a crystallinity, in particular determined by means of wide angle x-ray scattering (WAXS), from $\geq$ 65%, in particular 65 % to 99.9 %, preferably 75 % to 99 %, more preferably 80 % to 95 %.

Further, the crystallites of the permanently polarized hydroxyapatite may have a size, in particular determined by means of wide angle x-ray scattering (WAXS), from 20 nm to 500 nm, in particular 50 nm to 200 nm, preferably 70 nm to 100 nm. Preferably, in this context, the term "size" refers to an average diameter of the crystallites of the permanently polarized hydroxyapatite.

In a further embodiment of the invention, the permanently polarized hydroxyapatite has a proportion, based on the total weight of the catalyst, which is larger than a proportion of the brushite and/or brushite-like material, also based on the total weight of the catalyst.

In a further embodiment of the invention, the permanently polarized hydroxyapatite has a proportion of 50 % by weight to 99.9 % by weight, in particular 65 % by weight to 99.9 % by weight, in particular 75 % by weight to 99 % by weight, preferably 80 % by weight to 95 % by weight, in particular 85 % by weight to 90 % by weight, in particular 85 % by weight to 88 % by weight, based on the total weight of the catalyst.

With respect to further features and advantages of the permanently polarized hydroxyapatite as used according to the present invention, it is referred to the PCT application WO 2018/024727 A1, the content of which is incorporated hereby by explicit reference.

Further, the brushite and/or the brushite-like material may have a crystallinity, in particular determined by means of wide angle x-ray scattering (WAXS), from 65 % to 99.9 %, in particular 75 % to 99 %, preferably 80 % to 95 %.

Further, crystallites of the brushite and/or the brushite-like material may have a size, in particular determined by means of wide angle x-ray scattering (WAXS), from 20 nm to 500 nm, in particular 50 nm to 200 nm, preferably 70 nm to 100 nm. Preferably, in this context, the term "size" refers to an average diameter of the crystallites of the brushite and/or the brushite-like material.

In a further embodiment of the invention, the brushite and/or the brushite-like material has a proportion of > 0 % by weight to 50 % by weight, in particular 0.1 % by weight to 35 % by weight, in particular 1 % by weight to 25 % by weight, preferably 5 % by weight to 20 % by weight, in particular 10 % by weight to 15 % by weight, in particular 12 % by weight to 15 % by weight, based on the total weight of the catalyst.

Further, the catalyst may have a total catalytic activity ratio of the permanently polarized hydroxyapatite to the brushite and/or the brushite-like material of 0.5 : 2, in particular 0.75 : 1.5, preferably 0.8 : 1.25, with respect to the sum of yield of all amino acids of an amino acid mixture. The total catalytic activity may be preferably determined by means of $^1$H-NMR (nuclear magnetic resonance) spectroscopy. For that purpose, the areas of the peaks in the $^1$H-NMR spectrum are preferably normalized according to the number of the protons for each product obtained.

Further, the catalyst may in addition comprise amorphous calcium phosphate, in particular in the form of a further phase of the catalyst. The amorphous calcium phosphate may have a proportion of amorphous calcium phosphate of > 0 % by weight to 15 % by weight, in particular from > 0 % by weight to 10 % by weight, preferably > 0 % by weight to 5 % by weight, based on the total weight of the catalyst.

Alternatively, the catalyst may be free of amorphous calcium phosphate.

Further, the catalyst may in addition comprise tricalcium phosphate, in particular β-tricalcium phosphate. The tricalcium phosphate, in particular β-tricalcium phosphate, may be in the form of a further phase of the catalyst. The tricalcium phosphate, in particular β-tricalcium phosphate, may have a proportion of tricalcium phosphate, in particular β-tricalcium phosphate, of > 0 % by weight to 15 % by weight, in particular > 0 % by weight to 10 % by weight, preferably > 0 % by weight to 5 % by weight, based on the total weight of the composition or material.

Alternatively, the catalyst may be free of tricalcium phosphate, in particular β-tricalcium phosphate.

Further, the catalyst may have a bulk resistance of $10^7$ Ω cm$^2$ to $10^5$ Ω cm$^2$, in particular $10^6$ Ω cm$^2$ to $10^5$ Ω cm$^2$, preferably $10^5$ Ω cm$^2$. In particular, the bulk resistance may increase (only) from 4 % to 33 %, in particular 4 % to 63 %, preferably 4 % after 3 months. The term "bulk resistance" as used according to the present invention means resistance to the electron transfer and may be determined by means of electrochemical impedance spectroscopy.

Further, the catalyst may have a surface capacitance which decreases less than 15 %, in particular less than 8 %, after 3 months. Preferably, the catalyst may have a surface capacitance which decreases from 0 % or > 0 % to 15 %, more preferably from 0 % or > 0 % to 5 %, after 3 months. The term "surface capacitance" as used according to the present invention means capacitance attributed to surface changes of hydroxyapatite induced by a thermal polarization process and may be determined by means of electrochemical impedance spectroscopy.

Further, the catalyst may be in the form of particles. The particles may have a diameter, preferably mean diameter, in particular determined by means of wide angle x-ray scattering (WAXS), of 20 nm to 500 nm, in particular 50 nm to 200 nm, preferably 70 nm to 100 nm.

Further, the catalyst may be in the form of a powder, in particular having particles as mentioned in the preceding paragraph.

Further, the catalyst may be in the form of a shaped body. The shaped body may have a polygonal, for example triangular, quadratic or rectangular, pentagonal, hexagonal, heptagonal, octagonal or nonagonal, cross-section or a corner-less, in particular circular, oval-shaped or elliptical, cross-section.

In particular, the catalyst or the shaped body may be in the form of a disc, plate, cone (conus) or cylinder.

Further, the catalyst or shaped body may have a thickness of > 0 cm to 10 cm, in particular > 0 cm to 1 cm, preferably > 0 mm to 2 mm.

Further, the catalyst may be in the form of a coating.

Further, the catalyst may comprise an additional catalytically effective material, in particular in the form of an inorganic catalytically effective material, preferably in the form of an inorganic photocatalytically effective material. More specifically, the catalyst may be at least partially, in particular only partially or completely, coated with the additional catalytically effective material. The additional catalytically effective material may be a catalyst such as $TiO_2$, $MgO_2$, $MnO_2$ or combinations thereof.

Further, the catalyst may be at least partially, in particular only partially or completely, coated with aminotris(methylenephosphonic acid) and/or zirconium oxychloride ($ZrOCl_2$) and/or zirconia ($ZrO_2$). More specifically, the catalyst may have a three-layered coating, in particular wherein the three-layered coating may be composed of two layers of aminotris(methylenephosphonic acid) and a layer of zirconium oxychloride ($ZrOCl_2$) or zirconia ($ZrO_2$), wherein the layer of zirconium oxychloride is arranged or sandwiched between the two layers of aminotris(methylenephosphonic acid).

Preferably, the catalyst is obtained or obtainable by a process comprising the following steps:

(a) providing a sample of hydroxyapatite, in particular natural or synthetic hydroxyapatite, and/or amorphous calcium phosphate,

(b) sintering the sample of hydroxyapatite and/or amorphous calcium phosphate provided in step (a),

(c) applying a constant or variable DC voltage between 250 V and 2500 V, in particular for at least 1 min and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
applying an equivalent electric field between 1.49 kV/cm and 15 kV/cm, in particular for at least 1 min and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
applying an electrostatic discharge between 2500 V and 1500000 V, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) or
applying an equivalent electric field between 148.9 kV/cm and 8928 kV/cm, in particular for > 0 min to 24 h and/or at a temperature between 900 °C and 1200 °C, in particular from 1000 °C to 1200°C, to the sintered sample of hydroxyapatite and/or amorphous calcium phosphate obtained in step (b) or to a shaped body obtained from the sintered sample of hydroxyapatite and/or amorphous calcium phosphate obtained in step (b) and

(d) cooling the sample obtained in step (c) maintaining the DC voltage or the equivalent electric field or cooling the sample obtained in step (c) maintaining or without maintaining the electrostatic discharge or the equivalent electric field,

wherein, for performing step (c), the sintered sample of hydroxyapatite and/or amorphous calcium phosphate obtained in step (b) or the shaped body obtained from the sintered sample of hydroxyapatite and/or amorphous calcium phosphate obtained in step (b) is arranged between a positive electrode and a negative electrode, which are used for applying the constant or variable DC voltage, equivalent electric field or electrostatic discharge during step (c), such that the sintered sample of hydroxyapatite and/or amorphous calcium phosphate obtained in step (b) or the shaped body obtained from the sintered sample of hydroxyapatite and/or amorphous calcium phosphate obtained in step (b) is spaced from one of the two electrodes, preferably from the positive electrode, i.e. such that the sintered sample of hydroxyapatite and/or amorphous calcium phosphate obtained in step (b) or the shaped body obtained from the sintered sample of hydroxyapatite and/or amorphous calcium phosphate obtained in step (b) has a distance to one of the two electrodes, preferably to the positive electrode.

Preferably, for performing step (c), the sintered sample of hydroxyapatite and/or amorphous calcium phosphate obtained in step (b) or the shaped body obtained from the sintered sample of hydroxyapatite and/or amorphous calcium phosphate obtained in step (b) is arranged between the positive electrode and the negative electrode, which are used for applying the constant or variable DC voltage, equivalent electric field or electrostatic discharge during step (c), such that the sintered sample of hydroxyapatite and/or amorphous calcium phosphate obtained in step (b) or the shaped body obtained from the sintered sample of hydroxyapatite and/or amorphous calcium phosphate obtained in step (b) is spaced only from one of the two electrodes, preferably only from the positive electrode, i.e. such that the sintered sample of hydroxyapatite and/or amorphous calcium phosphate obtained in step (b) or the shaped body obtained from the sintered sample of hydroxyapatite and/or amorphous calcium phosphate obtained in step (b) has a distance only to one of the two electrodes, preferably only to the positive electrode. In other words, the sintered sample of hydroxyapatite and/or amorphous calcium phosphate obtained in step (b) or the shaped body obtained from the sintered sample of hydroxyapatite and/or amorphous calcium phosphate obtained in step (b) is preferably left in contact with the other of the two electrodes, more preferably with the negative electrode.

Further, the sintered sample of hydroxyapatite and/or amorphous calcium phosphate obtained in step (b) or the shaped body obtained from the sintered sample of hydroxyapatite and/or amorphous calcium phosphate obtained in step (b) is spaced from the one of the two electrodes, preferably from the positive electrode, in a distance from > 0 cm to 10 cm, in particular > 0 cm to 5 cm, preferably > 0 cm to 0.1 cm.

The electrodes, in particular the positive electrode and/or the negative electrode, can be of different shapes. The electrodes, in particular the positive electrode and/or the negative electrode, may have a polygonal cross-section, for example triangular, quadratic or rectangular, pentagonal, hexagonal, heptagonal, octagonal or nonagonal cross-section, or a corner-less, in particular circular, oval-shaped or elliptical, cross-section. Further, the electrodes, in particular the positive electrode and/or the negative electrode, may be in the form of a plate or disc, cone (conus) or a cylinder. Further, the electrodes, in particular the positive electrode and/or the negative electrode, can be made of steel, in particular stainless steel.

Further, the electrodes, i.e. the positive electrode and the negative electrode, may have a mutual distance from 0.01 mm to 10 cm, in particular 0.01 mm to 5 cm, preferably 0.01 mm to 1 mm.

Preferably, the step (a) comprises

- subjecting a suspension, in particular an aqueous suspension, preferably an aqueous-alcoholic suspension, containing calcium phosphate to a hydrothermal treatment.

The term "aqueous-alcoholic suspension" as used according to the present invention means a suspension containing water and an alcohol, in particular ethanol, as a solvent or solvent mixture.

Preferably, the suspension containing calcium phosphate is obtained by using an aqueous solution containing ammonium phosphate dibasic (diammonium hydrogen phosphate, $(NH_4)_2HPO_4$) and an alcoholic, in particular ethanolic, solution containing calcium nitride $(Ca(NO_3)_2)$. More preferably, the pH of the calcium nitride is adjusted at 10 to 12, in particular 10 to 11.5, more preferably 10.5 to 11. More preferably, the aqueous solution containing ammonium phosphate dibasic is added to the alcoholic, in particular ethanolic, solution containing calcium nitride. Further, the resulting mixture is preferably stirred, in particular for > 0 h to 24 h, at room temperature to form the suspension containing calcium phosphate. The latter may also be termed as "aging" of the reaction mixture.

Further, the hydrothermal treatment is preferably carried out in an autoclave, in particular under a pressure of > 0 bar to 200 bar, in particular > 0 bar to 50 bar, preferably > 0 bar to 20 bar.

Further, the hydrothermal treatment is preferably carried out at a temperature of 50 °C to 240 °C, in particular 100 °C to 240 °C, more preferably 110 °C to 240 °C, more preferably 120 °C to 240 °C, more preferably 130 °C to 240 °C, more preferably 140 °C to 240 °C, especially preferably 150 °C to 240 °C.

Further, the hydrothermal treatment is preferably carried out for > 0 h to 48 h, in particular > 0 h to 24 h, preferably 12 h to 24 h.

In particular, the hydrothermal treatment may be carried out by autoclaving the afore-mentioned reaction mixture, in particular after aging, at 150 °C for 24 h.

The step (a) may further comprise

- cooling the hydrothermally treated suspension containing calcium phosphate to form a precipitate. In particular, the hydrothermally treated suspension containing calcium phosphate may be cooled to a temperature of 0 °C to 90 °C, in particular 10 °C to 75 °C, preferably of 25 °C.

The step (a) may further comprise

- separating the precipitate obtained by cooling the hydrothermally treated suspension containing calcium phosphate, in particular by means of centrifugation and/or filtration.

The step (a) may further comprise

- washing the separated precipitate obtained by cooling the hydrothermally treated suspension containing calcium phosphate, in particular using water and/or an alcohol, in particular ethanol.

The step (a) may further comprise

- freeze-drying the separated and optionally washed precipitate obtained by cooling the hydrothermally treated suspension containing calcium phosphate to produce hydroxyapatite, in particular synthetic hydroxyapatite. The freeze-drying may be carried out for 1 day to 4 days, in particular for 2 days to 3 days, preferably for 3 days.

Further, the sample of hydroxyapatite in step (a) may be in particular a sample of crystalline hydroxyapatite.

Further, the above-mentioned step (b) may be carried out at a temperature between 700 °C and 1150 °C, in particular between 800 °C and 1100 °C, in particular at 1000 °C.

Further, the step (b) may be carried out repeatedly. Thus, the crystal structure of the hydroxyapatite may be advantageously refined.

Further, the process according to the present invention preferably comprises between the step (b) and the step (c) a further step (bc)

- shaping, in particular pressing, the sintered sample of hydroxyapatite and/or amorphous calcium phosphate obtained in step (b) to form the shaped body.

Accordingly, the shaped body may be preferably obtained from the sintered sample of hydroxyapatite and/or amorphous calcium phosphate obtained in step (b) by shaping, in particular pressing, said sample.

In particular, the step (bc) may be carried out under a pressure of 1 MPa to 1000 MPa, in particular 100 MPa to 800 MPa, preferably 600 MPa to 700 MPa. Further, the step (bc) may be carried out for 1 min to 90 min, in particular 5 min to 50 min, preferably 10 min to 30 min.

The shaped body may have a polygonal, for example triangular, quadratic or rectangular, pentagonal, hexagonal, heptagonal, octagonal or nonagonal, or a corner-less, in particular circular, oval-shaped or elliptical, cross-section. Further, the shaped body may have a thickness of 0.1 cm to 10 cm, in particular 0.1 cm to 5 cm, preferably 0.5 cm to 2 cm.

Preferably, the shaped body is in the form of a disc, plate, cone (conus) or cylinder.

Further, the constant or variable DC voltage or the equivalent electric field may be applied in the above-mentioned step (c) for 1 h to 24 h, in particular 0.1 h to 10 h, in particular 1 h.

Further, the DC voltage applied in the above-mentioned step (c) is preferably 500 V, which is equivalent to a constant electric field of 3 kV/cm.

Further, the equivalent electric field applied in the above-mentioned step (c) is preferably 3 kV/cm.

Further, the temperature in step (c) is preferably at least 900 °C, more preferably at least 1000 °C, in particular 1000 °C. Preferably, the temperature in step (c) is 900 °C to 1200 °C, in particular 1000 °C to 1200°C.

Further, the above-mentioned step (d) may be carried out by cooling the sample obtained in step (c) to room temperature.

Further, the above-mentioned step (d) may be carried out for 1 min to 72 h, in particular 15 min to 5 h, preferably 15 min to 2 h.

Preferably, for performing the above-mentioned step (c), the sample obtained in step (b) or the shaped sample thereof is spaced > 0 cm to 10 cm, in particular 0.1 cm to 7 cm, preferably 1 cm to 5 cm, from the one of the two electrodes, preferably from the positive electrode. Preferably, the sample obtained in step (b) or the shaped sample thereof is left in physical contact with the other of the two electrodes, preferably with the negative electrode.

In a further embodiment of the invention, the contacting step is carried out in the presence of liquid water and/or water vapor. In other words, in a further embodiment of the invention, the water is in liquid form and/or vapor form, for carrying out the contacting step.

In a further embodiment of the invention, the contacting step is carried out with a volumetric ratio of the catalyst to the water, in particular liquid water and/or water vapor, of 1000 : 1 to 0.01 : 1, in particular 500 : 1 to 0.05 : 1, preferably 300 : 1 to 350 : 1.

In a further embodiment of the invention, the contacting step is carried out with a volumetric ratio of the carbon dioxide to the methane to the nitrogen of 100 :

1 : 1 or 1 : 100 : 1 or 1 : 1 : 100, in particular 10 : 1 : 1 or 1 : 10 : 1 or 1 : 1 : 10, preferably 1 : 1 : 1.

In a further embodiment of the invention, the contacting step is carried out under a total pressure of > 0 bar to 250 bar, in particular > 0 bar to 100 bar, preferably > 0 bar to 10 bar.

The term "total pressure" as used according to the present invention refers to the sum of each gas partial pressure of the gas mixture, preferably at room temperature.

In a further embodiment of the invention, the contacting step is carried out under a partial pressure of the carbon dioxide of > 0 bar to 100 bar, in particular > 0 bar to 50 bar, preferably > 0 bar to 5 bar and/or under a partial pressure of the methane of > 0 bar to 100 bar, in particular > 0 bar to 50 bar, preferably > 0 bar to 10 bar and/or under a partial pressure of the nitrogen of > 0 bar to 100 bar, in particular > 0 bar to 50 bar, preferably > 0 bar to 10 bar.

In a further embodiment of the invention, the contacting step is carried out with a molar ratio of the carbon dioxide to the catalyst of > 0 to 0.5, in particular 0.2 to 0.5, preferably 0.3 to 0.5, and/or with a molar ratio of the methane to the catalyst of > 0 to 0.5, in particular 0.2 to 0.5, preferably 0.3 to 0.5 and/or with a molar ratio of the nitrogen to the catalyst of > 0 to 0.5, in particular 0.2 to 0.5, preferably 0.3 to 0.5.

Further, the contacting step may carried out by using an uncoated form of the catalyst, i.e. by using the catalyst lacking any coating.

Alternatively, the contacting step may be carried out by using a coated form of the catalyst. In partiuclar, the contacting step may be carried out by using the catalyst, wherein the catalyst is coated with an inorganic photocatalytically effective material such as $TiO_2$, $MgO_2$, $MnO_2$ or combinations thereof. More specifically, the contacting step may be carried out by using the catalyst, wherein the catalyst has a three-layered coating, in particular wherein the three-layered coating may be composed of two layers of aminotris(methylenephosphonic acid) and a layer of zirconium oxychloride ($ZrOCl_2$) or zirconia ZrO2, wherein the layer of zirconium oxychloride is arranged or sandwiched between the two layers of aminotris(methylenephosphonic acid). By using this coated form of the catalyst, the efficiency of the reaction can be advantageously increased.

In a further embodiment of the invention, the contacting step is carried out under UV (ultraviolet) irradiation or UV-Vis (ultraviolet-visible) irradiation. In particular, the contacting step may be carried out under UV irradiation or UV-Vis irradiation having a wavelength from 200 nm to 850 nm, in particular 240 nm to 400 nm, preferably 250 nm to 260 nm, more preferably of 253.7 nm. Particularly, the contacting step may be carried out under UV irradiation having a wavelength from 200 nm to 280 nm, in particular 240 nm to 270 nm, preferably 250 nm to 260 nm, more preferably of 253.7 nm. Preferably, the catalyst is directly exposed to or irradiated with the UV irradiation or UV-Vis irradiation. Advantageously, the UV irradiation or UV-Vis irradiation are/is provided by a suitable UV source and/or Vis source, for example UV lamp and/or Vis lamp.

In a further embodiment of the invention, the contacting step is carried out under UV (ultraviolet) irradiation or UV-Vis (ultraviolet-visible) irradiation having a irradiance from 0.1 $W/m^2$ to 200 $W/m^2$, in particular 1 $W/m^2$ to 50 $W/m^2$, preferably 2 $W/m^2$ to 10 $W/m^2$, more preferably of 3 $W/m^2$.

In a further embodiment of the invention, the contacting step is carried out at a temperature of 25 °C to 250 °C, in particular 95 °C to 140 °C, preferably of 95 °C.

Further, the contacting step may be carried out without UV irradiation and at a temperature of 25 °C to 250 °C, in particular 95 °C to 140 °C, preferably of 140 °C.

Further, the contacting step may be carried out continuously or discontinuously, in particular as a batch process.

Further, the contacting step may be carried out for 0.0001 h to 120 h, in particular 24 h to 72 h, preferably 48 h to 72 h.

Further, the contacting step may be carried out by using air, in particular traffic contaminated air, as the gas mixture. Thus, it is possible to synthesize amino acids, in particular glycine and/or alanine, as valuable compounds and concurrently to remove carbon dioxide from air, in particular traffic contaminated air.

Preferably, the process according to the present invention comprises a further step

- isolating and/or separating and/or purifying the amino acids, in particular glycine and/or alanine, obtained during or in the contacting step.

The above further step is preferably carried out by dissolving and extracting the catalyst and/or by extracting a supernatant formed during or in the contacting step.

Preferably, the process according to the present invention is used for producing or synthesizing amino acids, in particular natural amino acids, preferably glycine and/or alanine.

Further, the present invention relates to the use of the process according to the present invention for removing harmful gases such as carbon dioxide, carbon monoxide, methane or mixtures thereof, in particular from an atmosphere, preferably from air, i.e. atmosphere of Earth. In particular, the present invention relates to the use of the process according to the present invention for removing harmful gases such as carbon dioxide, carbon monoxide, methane or mixtures thereof, from polluted or contaminated air such as traffic contaminated air.

The term "air" or "atmosphere of Earth" as used according to the present invention means a layer of gases retained by Earth's gravity, surrounding the planet's Earth and forming its planetary atmosphere.

With respect to further features and advantages of the uses described in the preceding paragraphs, reference is made in its entirety to the previous description.

Finally, the present invention relates to the use of a process comprising the step of

- contacting a gas mixture comprising or consisting of carbon dioxide, methane and nitrogen in presence of water with a catalyst, wherein the catalyst comprises or consists of permanently polarized hydroxyapatite and brushite or wherein the catalyst comprises or consists of permanently polarized hydroxyapatite and a brushite-like material or wherein the catalyst comprises or consists of permanently polarized hydroxyapatite, brushite and a brushite-like material,

for the production or synthesis, in particular selective production or synthesis, of amino acids, in particular natural amino acids, preferably glycine and/or alanine.

With respect to further features and advantages of the uses described in the preceding paragraphs, reference is made in its entirety to the previous description.

Further features and advantages of the invention will become clear from the following examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

BRIEF DESCRIPTION OF THE FIGURES

In the figures, the following is schematically displayed:

**Figure 1.** (a) WAXS diffraction patterns in the characteristic $2\theta$ range from 20° to 60° for multiphasic HAp-brushite catalysts (hereafter named C-2); (b) Raman spectra of the $PO_4^{3-}$ internal modes ($V_1$, $V_2$, $V_3$ and $V_4$) for C-2. (c) WAXS diffraction patterns in the characteristic $2\theta$ range from 20° to 60° and (d) Raman spectra of the $PO_4^{3-}$ internal modes ($V_1$, $V_2$, $V_3$ and $V_4$) for single-phase hydroxyapatite (HAp) catalysts (hereafter named C-1) and C-2. Characteristic reflections and vibrations for HAp and brushite are labelled in each sub-figure.

**Figure 2.** Stacked Raman spectra obtained from a $7 \times 4$ array with a spacing of 1 $\mu$m for the C-1 and C-2 catalysts. Scale bar refers to the relative intensity of the peaks.

**Figure 3.** In-depth analyses of Raman spectra of C-1 and C-2 of the zones of interest: (a) v1 principal mode; (b) lattice modes; and (c) O-H stretching vibrational mode in the region of 3500 to 3700 cm$^{-1}$ (acquired with a 532 nm laser). Characteristic Raman vibrations for HAp and brushite are labelled in black and green, respectively.

**Figure 4.** XPS spectra of Ca 2p obtained for C-1 and C-2 catalysts.

**Figure 5.** (a) Unit cell of HAp crystalline structure; (b) OH$^-$ channels along the c axis of HAp crystalline structure.

**Figure 6.** (a) Unit cell of brushite; (b) unit cell of monetite.

**Figure 7.** (a) and (b) Crystalline structure of brushite. The atoms involved in the hydrogen bonds have been labeled.

**Figure 8.** Raman spectra of (a) crystalline hydroxyapatite (hereafter named cHAp)($T_h$) and (b) thermally stimulated polarized crystalline (hereafter named cHAp/tsp)($T_h$) prepared using different hydrothermal temperatures for 24 h.

**Figure 9.** Raman spectra in the (a) 770-910 cm$^{-1}$ and (b) 100-350 cm$^{-1}$ intervals of cHAp(150 °C) and cHAp/tsp($T_h \geq$ 100 °C).

**Figure 10.** (a) Raman spectra of cHAp(150 °C) and cHAp/tsp(150 °C) prepared using different times for the HT: 10 h and 24 h. (b) Raman spectra of cHAp/tsp(150 °C) prepared by applying different DC voltages to cHAp(150 °C). (c) Raman spectra of cHAp/tsp(150 °) prepared by applying a voltage of 500 V and maintaining the steel electrodes in contact or not with the cHAp(150 °C) samples. cHAp(150 °C) were prepared applying the HT for 24 h in both (b) and (c).

**Figure 11.** (a) Raman spectra at different depths of cHAp/tsp(150 °C) obtained applying the HT for 24 h and a polarizing voltage of 500 V at 1000 °C. (b) Magnification of the spectra displayed in (a) for the 100-350 cm$^{-1}$ region.

**Figure 12.** Low magnification SEM micrographs of cHAp($T_h$) with $T_h$= 50, 100, 150, 200 and 240 °C. In all cases the HT was applied for 24 h. High magnification micrographs of cHAp(150 °C) are also displayed.

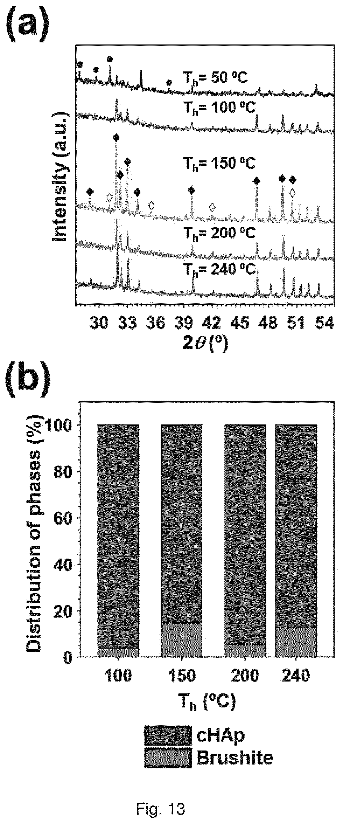

**Figure 13.** (a) X-ray diffraction patterns corresponding to cHAp($T_h$) with $T_h$= 50, 100, 150, 200 and 240 °C. In all cases the HT was applied for 24 h. Reflections attributed to cHAp and brushite are marked by filled diamonds and empty diamonds, respectively, in the diffractogram of cHAp(150 °C), while those associated with βTCP (β-tricalcium phosphate) are indicated by filled circles in the diffractogram of cHAp(50 °C). (b) Distribution of cHAp and brushite phases in cHAp($T_h$) with $T_h \geq$ 100 °C. This was obtained from the (211) reflection of cHAp and the (141) reflection of brushite.

**Figure 14.** For sample obtained after reaction (95 °C and 48 h) using a chamber pressure of 6 bar (i.e. 2 bar of each feeding reaction gas) and by the catalyst prepared using cHAp(150 °C): (a) $^1$H NMR spectrum of the solution obtained after extraction of the amino acids from the catalyst by dissolving the sample in deuterated water containing 100 mM of HCl and 50 mM of NaCl; and (b) solid state $^{13}$C NMR spectra of the catalyst with the synthesized amino acids.

**Figure 15.** Yield (in % per cm$^2$ of catalyst) of alanine and glycine in the electrophotocatalytic fixation of $N_2$, $CO_2$ and $CH_4$ as a function of $T_h$. In all cases reactions (in triplicate) were performed at 6 bar and 95 °C for 48 h.

**Figure 16.** $^{31}$P-NMR spectrum of permanently polarized hydroxyapatite.

EXPERIMENTAL SECTION

1. Materials

Calcium nitrate Ca(NO$_3$)$_2$, diammomium hydrogen phosphate [(NH$_4$)$_2$HPO$_4$; purity > 99.0%], ammonium hydroxide solution 30% [NH$_4$OH; purity: 28-30% w/w], zirconyl chloride (ZC; ZrOCl$_2$·8H$_2$O) and aminotris(methylene phosphonic acid) (ATMP) were purchased from Sigma Aldrich. Ethanol (purity > 99.5%) was purchased from Scharlab. All experiments were performed with milli-Q water. $N_2$, $CH_4$ and $CO_2$ gases with a purity of >99.995% were purchased from Messer.

2. Synthesis of crystalline hydroxyapatite (cHAp)

15 mL of 500 mM (NH$_4$)$_2$HPO$_4$ in de-ionized water (pH adjusted to 10.5 ± 0.2 with ammonium hydroxide) were added drop-wise (2 mL/min) and under gentle agitation (100 rpm) to 25 mL of 500 mM Ca(NO$_3$)$_2$ in ethanol. The mixture was stirred for 1 h (100 rpm) at room temperature resulting in a suspension. Hydrothermal (HT) treatment was applied to the suspension with temperature ($T_h$) ranging from 50 °C to 240 °C using an autoclave Digestec DAB-2 for 24 h unless otherwise is specified. The autoclave was allowed to cool down before opening. The white precipitates were separated by centrifugation and washed sequentially at 8000 rpm for 5 minutes with water and a 60/40 v/v ethanol/water mixture (twice). After freeze-drying for 3 days the powder obtained was sintered at 1000 °C during 2 h at an air atmosphere, in particular using a Carbolite ELF11/6W/301 furnace. Hereafter, samples obtained using this procedure have been denoted cHAp($T_h$), where $T_h$ refers to the temperature used for the HT of hydroxyapatite (HAp).

3. Thermally stimulated polarization process (TSP)

According to a first approach, 150 mg of sintered cHAp powder were uniaxially pressed at 620 MPa for 10 minutes (i.e. 5 tons of applied weight) to obtain a disc of 10 mm of diameter and 1 mm of thickness. The disc was placed in between two stainless steel (AISI 304) plates separated at 4 cm and heated at 1000 °C in air atmosphere. Then, a DC voltage of 100 V, 500 V or 1000 V was applied during 1 h. Hereafter, samples obtained by applying the TSP process to cHAp($T_h$) are denoted cHAp/tsp($T_h$), the applied voltage being explicitly indicated in each case.

According to a second approach, catalytic activation was (also) successfully achieved applying the thermal stimulation polarization (TSP) treatment, which consisted in exposing the HAp disks at 500 V and 1000 °C for 1 h. For obtaining single-phase HAp catalysts (hereafter named C-1), the electrodes (two stainless steel AISI 304 plates) were placed in contact with the HAp disk. Instead, multiphasic HAp-brushite catalysts (hereafter named C-2) were attained by separating the positive electrode 4 cm from the HAp disk, which was left in contact with the negative electrode.

4. Characterization

Structural characterization studies were conducted using X-ray photoelectron spectroscopy (XPS), micro-Raman spectroscopy and wide angle X-ray scattering (WAXS). From WAXS spectra, crystallinity ($X_c$) and crystallite sizes $L_{hkl}$ were determined.

Further, the structural fingerprint of the samples was studied using the inVia Qontor confocal Raman microscope (Renishaw), equipped with a Renishaw Centrus 2957T2 detector. All measurements were performed with a 532 and a 785 nm laser. In order to achieve representative results, all the spectra presented in this study are the result of the average of a 105x90 $\mu$m grid with 42 points. Depth profiles were also obtained using the same equipment.

Morphological characterization has been performed by scanning electron microscopy (SEM) using a Focused Ion Beam Zeiss Neon40 microscope equipped with a SEM GEMINI column with a Shottky field emission. Samples were sputter-coated with a thin layer of carbon to prevent sample charging problems.

As mentioned, crystallinity ($\chi_c$) was obtained by wide angle X-ray scattering (WAXS) using a Brucker D8 Advance model with Bragg-Brentano $2\theta$ configuration and Cu K$_\alpha$ radiation ($\lambda$ = 0.1542 nm). A one-dimensional Lynx Eye detector was employed. Measurements were performed in a $2\theta$ range of 20° - 60° in steps of 0.02°, and scan speed of 2 s. The $\chi_c$ value was obtained using the following expression:

$$\chi_c = 1 - \frac{V_{112/300}}{I_{300}} \quad (1)$$

where $I_{300}$ is the intensity of the (300) reflection and $V_{112/300}$ is the intensity of the hollow between the (112) and (300) reflections. The crystallite size, $L_{hkl}$, was calculated using the Debye-Scherrer equation

$$L_{hkl} = \frac{0.9 \cdot \lambda}{B \cdot \cos\theta_{hkl}} \quad (2)$$

where $\lambda$ is the wavelength of the monochromatic X-ray beam, $B$ is the full width at half maximum of the peak at the maximum intensity, and $\theta_{hkl}$ is the peak diffraction angle that satisfies the Bragg's law for the (hkl) plane.

## 5. Carbon Fixation

The reaction was carried out in an inert reactor chamber with a 3 bar $CO_2$ and 3 bar $CH_4$ atmosphere at 95 °C for 72 h under UV irradiation (GPH265T5L4, 253.7 nm). Before sealing, 0.5 mL of de-ionized liquid water were added to the reactor. The products of the reaction from the catalyst surface were analyzed by $^1$H-NMR spectroscopy (Bruker Avance III-400). Yields of the reaction were obtained using commercial products with controlled concentration as a reference.

## 6. Effect of TSP treatment on the coexistent brushite phase

Figure 1(a), which compares the WAXS spectra recorded for C-1 and C-2, reveals the unambiguous presence of highly crystalline HAp in both catalysts. Thus, the peaks identified at $2\theta$ = 31.8°, 32.2°, and 33.0° correspond to the (211), (112) and (300) reflections of HAp, respectively (JCPDS card number 9-0432). The most significant peaks of the co-existent brushite appear at $2\theta$ = 29°, 31°, 35°, 42°, and 51°, which have been attributed to the (141), (22$\overline{1}$), (121), (15$\overline{2}$) and (14$\overline{3}$) reflections, respectively (JCPDS card number 72-0713). As it can be seen, although the (22$\overline{1}$), $2\theta$ = 31.0°, is the only distinguishable reflection for brushite in figure 1a, comparison of the relative intensities obtained for C-1 and C-2 supports the presence of this co-existing apatitic phase in the latter. One of the most relevant differences is observed at $2\theta$ = 51.3°, which can be attributed to the (410) reflection of HAp or to the (14$\overline{3}$) of brushite. For C-1 the intensity of the peak at $2\theta$ = 51.3° relative to the one at $2\theta$ = 52.1°, which corresponds to the (402) HAp reflection, is lower than one ($I_{2\theta=51.0}$ / $I_{2\theta=52.1}$ = 0.96), indicating that the intensity of the former peak is slightly lower than that of the latter. For C-2, $I_{2\theta=51.0}$ / $I_{2\theta=52.1}$ increases to $I_{51/52.1}$ = 1.14, evidencing that the peak at $2\theta$ = 51.3° becomes more intense than the one at $2\theta$ = 52.1°.

The (112) and (300) peaks were also used to determine the crystallinity ($\chi_c$; Eq S1), whereas the (211) reflection was used to calculate the crystallite size ($L_{211}$; Eq S2). The crystallinity is very high and similar for the two catalysts, $\chi_c$ = 0.95 $\pm$ 0.03 and 0.92 $\pm$ 0.03 for C1- and C-2, respectively. Crystallite sizes are also comparable for the two catalysts, the obtained values ($L_{211}$ = 75.2 $\pm$ 2.4 and 82.7 $\pm$ 3.72 nm for C-1 and C-2, respectively), being in agreement with those reported in the literature. Overall, these observations indicate that the differences applied during the TSP treatment do not affect the predominant HAp phase.

Raman studies on C-1 and C-2 catalysts are presented in figure 1(b). The spectra of apatitic phases are mainly dominated by their characteristic P-O vibrations. The four characteristic regions of HAp, which correspond to the $PO_4^{3-}$ internal modes, can be seen in the spectra of the catalysts, being: $V_1$= 962 cm$^{-1}$, $V_2$= 400-900 cm$^{-1}$, $V_3$= 570-625cm$^{-1}$ and $V_4$= 1020-1095 cm$^{-1}$. Both samples present a slight splitting of the $V_1$ mode in another two peaks at 970 and 949 cm$^{-1}$, which has attributed to the different P-O stretching vibrations of the three crystallographic non-equivalent $PO_4^{3-}$ tetrahedra found in the β-tricaicium phosphate (TCP) apatitic phase. Accordingly with the literature, the presence of TCP is not related to the TSP treatment but to small variation in the conditions during the HT synthesis and the sintering applied afterwards. Moreover, previous studies showed that the TSP treatment increases the crystallintiy and reduces TCP phase by imposing crystallographic specific orientations. Besides, the fact that the two samples present approximately the same relative amount of TCP (i.e. the ratio of the intensities of the Raman shift at 970 cm$^{-1}$ and the main peak at 962 cm$^{-1}$, $I_{970/962}$, is 0.12 and 0.10 for C-1 and C-2 respectively) evidences that differences in the conditions applied during the TSP treatment are not the precursors of the TCP generation. However, the effect of separating the positive electrode from the mineral disc during the TSP treatment is clearly manifested in figure 1b. More specifically, the presence of brushite in C-2 sample is confirmed by the peaks at 878, 848 and 794 cm$^{-1}$, which correspond to the normal vibration mode of $HPO_4^{2-}$, the POH deformation mode and the POH rotation mode, respectively. Other differences found between the Raman spectra of C-1 and C-2 catalysts are discussed below.

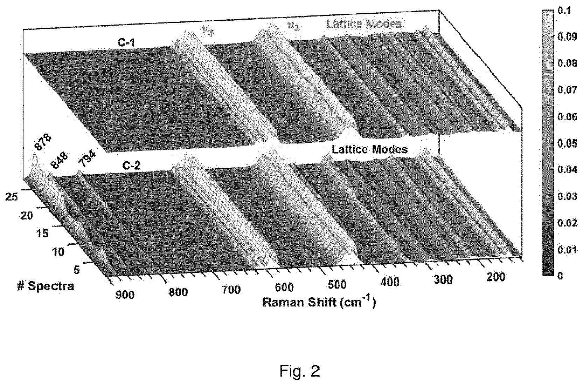

The evident presence of $HPO_4^{2-}$ and POH vibrations allow tracking the variations in the amount of brushite, depending on the TSP conditions. In this sense, the synergistic activity between the HAp and brushite phases is strongly dependent on their exposed surfaces and, therefore, characterization of their superficial distribution is of major interest. Figure 2 depicts 28 Raman spectra of C-1 and C-2 samples, which have recorded from a 7 x 4 array with a spacing of 1 $\mu$m. For clarity purposes, all spectra have been stacked together, the scale bar referring to the relative intensity of the peaks. As expected, the C-1 spectra do not show any trace of the brushite peaks aforementioned, in comparison with C-2 sample. Surprisingly, the 878 cm$^{-1}$ peak of C-2, which corresponds to the $HPO_4^{2-}$ normal vibration mode, presents variation in its relative intensity up to 90%, indicating that the coexisting brushite phase is distributed very heterogeneously. Results derived from figure 2 suggest that the geometry of the set-up used to apply the electric field plays an important role, defining the heterogeneity of the two co-existing phases. In order to support this conclusion, the inventors conducted additional experiments repeating the TSP treatment with the separated positive electrode. More specifically, the plate was removed, leaving only the cooper cable acting as the positive electrode. Examination of the recorded Raman spectra confirmed the dependence of the brushite phase with the geometry of the applied field. Not only the peaks $HPO_4^{2-}$ and POH vibration peaks appeared more intense (i.e. $I_{878/962} = 0.22$ and $I_{878/962} = 0.73$ for the C-2 catalysts prepared without and with plate at the positive electrode, respectively), but also the sample was more homogenous, the peak at 878 cm$^{-1}$ presenting a relative variation of ~21 % only. Overall, results demonstrate that the HAp/brushite ratio and heterogeneity of the catalyst can be regulated by varying the distance between the plates and the geometry of electric during the TSP treatment.

## 7. Structural Characterization of the C-2 catalyst

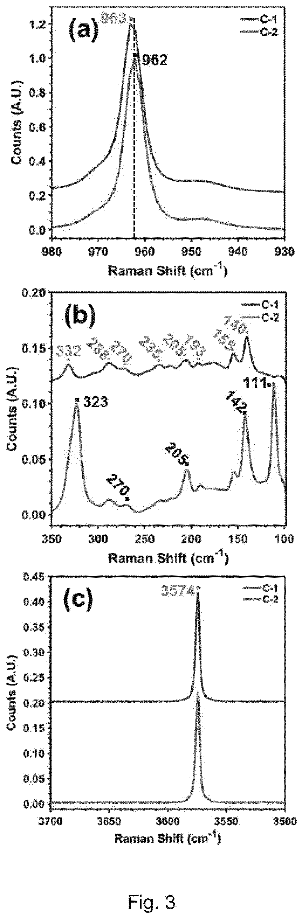

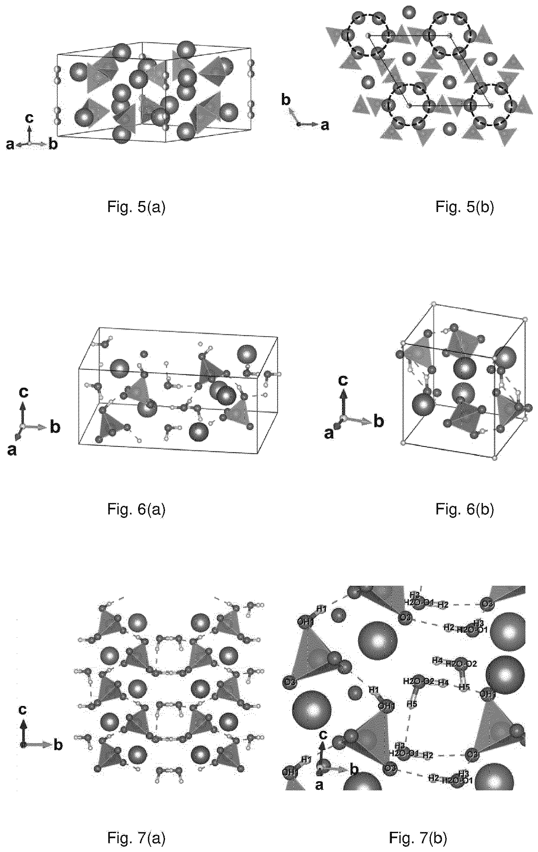

In order to understand the synergistic effects occurring in the brushite-containing catalyst, exhaustive structural characterization is crucial. In the case work, the inventor's efforts have been focused on discerning how brushite is integrated into the HAp crystal lattice as distortion on its boundaries, generating new active edge sites and increasing locally the electric conductivity. The most common polymorph of HAp is the hexagonal lattice (see figures 5 (a) and 5 (b)) with the space group $P6_3/m$ (a = b = 9.432 Å, c = 6.881 Å; $\alpha = \beta = 90°$, $\gamma = 120°$) $PO_4^{3-}$ groups are ordered in equivalent tetrahedra while $Ca^{2+}$ ions occupy two different crystallographic positions. In this crystalline phase, the OH$^-$ groups are ordered in columns along the c-axis but with disordered orientations because of electrostatic forces. Upon application of the TSP treatment, OH$^-$ groups tend to orient along the specific direction of the electric field, thus introducing crystallographic stress in the crystal lattice. This crystallographic stress is shown for C-1 in figure 3(a), which displays a magnification of the Raman spectrum in the region of the v1 principal mode. Whereas the main $V_1$ vibration mode of HAp of C-2 sample coincides with its theoretical value at 962 cm$^{-1}$, C-1 main peak is located at 963 cm$^{-1}$, reflecting the tensile strain of the $PO_4^{3-}$ tet-

rahedra. The fact that C-1 is the only sample presenting such shift indicates that the brushite found in the C-2 catalyst compensates the local tensile stress tension induced by re-arrangement of the OH$^-$ chains.

One of the most surprising aspects of the C-2 catalyst is that the attainment of the brushite phase is apparently contradictory with the fact that the TSP is carried out at high temperatures. At around 160 °C, brushite dehydrates to monetite ($CaHPO_4$) for further deprotonation to the $\gamma$, $\beta$ and $\alpha$ different forms of calcium pyrophosphate ($Ca_2P_2O_7$) when the temperature increases to 320, 700 and 1200 °C, respectively. The experimental evidence obtained from the $HPO_4^{2-}$ and the POH vibration modes at 878, 848 and 794 cm$^{-1}$, as well as the absence of the POP vibration at around 732 cm$^{-1}$, allow to discard the presence of $Ca_2P_2O_7$. The discrimination of brushite from monetite is clearly visualized by analyzing the lattice vibration modes since such structures crystallize in different space groups, as reflected in figures 6 (a) and 6 (b). Brushite consists of a monoclinic structure with an la space group symmetry (a = 5.799 Å, b = 15.126 Å, c = 6.184 Å; $\alpha = \gamma = 90°$, $\beta = 116.428°$), while monetite exhibits a triclinic unit cell with a $P\overline{1}$ space group symmetry (a = 6.916 Å, b = 6.619 Å, c = 6.946 Å; $\alpha = 96.180°$, $\beta = 103.82°$, $\gamma = 88.34°$). The lattice modes of the C-1 and C-2 catalysts are compared in figure 3(b). C-1 presents the characteristic lattice modes of HAp at: 140 and 155 cm$^{-1}$ (attributed to the transitional vibrations of $Ca_1 + Ca_2$ and $Ca_2$, respectively); 193 and 205 cm$^{-1}$ (translational vibration of $PO_4^{3-}$); 235 and 288 cm$^{-1}$ (librational vibrations of $PO_4^{3-}$); 270 cm$^{-1}$ (transitional vibration of $Ca_1$); and 332 cm$^{-1}$ (translational vibrations of OH$^-$).

In comparison with C-1, the spectrum recorded for C-2 displays much more intense peaks at 111, 142 and 270 cm$^{-1}$, which have been attributed to the contribution of the $Ca^{2+}$ unique crystallographic sites in brushite. Moreover, the $PO_4^{3-}$ translational mode at 205 cm$^{-1}$ is enhanced in C-2 with respect to C-1. Is it worth noting that, while the peak at 142 cm$^{-1}$ (assigned to the transitional vibrations of $Ca_1 + Ca_2$ of HAp and Ca of brushite) is much more intense and presents a red shift of 2 cm$^{-1}$ with respect to C-1, the peak at 155 cm$^{-1}$ remains unaltered, as it has been attributed to the HAp transitional vibration of $Ca_2$, which is inexistent in the brushite phase. The red shift of $Ca^{2+}$ transitional vibration from 140 cm$^{-1}$ in C-1 to 142 cm$^{-1}$ in C-2 confirms the presence of brushite in the latter, instead of monetite. Thus, due to group symmetries, the raman shift attributed to the $Ca^{2+}$ transitional vibration should be ordered as follows: monetite < HAp < brushite. Overall, the results shown in figure 3(a)-(b) not only confirms the presence of brushite but also highlights that the C-2 catalyst presents locally $Ca^{2+}$ with higher mobility and less tensile stress, which could cause a synergistic effect responsible of an enhance of its catalytic performance.

As shown in figures 7(a) and 7(b), the crystal structure of brushite can be understood as a layered system formed by zig-zag $Ca^{2+}$ and $PO_4^{3-}$ alternated chains parallel to the *a*-axis and growing along the c-axis. These chains are bond together along the *b*-axis through hydrogen bonds, provided by $H_2O$ molecules which form an intermediate layer. However, this water intermediate layer has not been detected in the Raman spectrum of C-2. Characteristic stretching modes for water should be detected in form of two duplets at 3539 and 3483 cm$^{-1}$ for $V_1$, and 3270 and 3163 cm$^{-1}$ for $V_2$.

Figure 3(c) shows the characteristic HAp peak corresponding to the OH$^-$ stretching mode of HAp at 3574 cm$^{-1}$. Moreover, neither the water liberations modes, which are typically located at 678 cm$^{-1}$, can be appreciated in figure 1(b).

Detailed analysis of the lattice modes in figure 3(b) revealed the presence of a strong new peak at 323 cm$^{-1}$ for C-2, which has been assigned to the translational vibration modes of OH$^-$ groups. This peak can be clearly deconvoluted into two different peaks located at 332 cm$^{-1}$, which matches the translational vibration of HAp obtained in the C-1 spectrum, and at 323 cm$^{-1}$. The fact that this new peak presents an important blue shift indicates the existence of a different crystallographic OH$^-$ with less mobility, and thus, somehow bonded. Due to their participation in hydrogen bonding interaction, $H_2O$ molecules of pure brushite are distorted from that of free molecules, generating two distinct crystallographic sites for water molecules (labeled in figure 7 as $H_2O$-O1 and $H_2O$-O2). The external layers are bond together via the O3$\cdots$H2 (of $H_2O$-O1) and OH1$\cdots$H4 (of $H_2O$-O2) hydrogen bond interactions, presenting short distances of 1.81 Å and 1.90 Å and almost linear angles of 167.3° and 175.7° respectively. Taking into account the theoretic aspects aforementioned and in agreement with the experimental results obtained, hydroxyl groups occupying the crystallographic positions of water molecules appear to be responsible for stabilizing the crystallographic brushite or brushite-like phase through hydrogen bonding interactions. The exchange of proton and hydroxyl groups at high temperatures, causing modifications in the crystalline structure of HAp, has been widely studied. However, substituting $H_2O$ molecules by OH$^-$ may cause other crystallographic distortions since the remaining water proton, which is hydrogen bonded to other oxygen of the lattice, is suppressed. For to case of $H_2O$-O2 water molecule this is not crucial for the stability of brushite, as H5 is weakly bonded to the $H_2O$-O1 water molecule, with a H5$\cdots H_2O$-O1 distance of 2.16 Å. Accordingly, the alternating $Ca^{2+}$ and $PO_4^{3-}$ ions form weaker interaction, and thus have more mobility. This hypothesis is supported by the drastic increment in the intensity of the lattice modes for such ions in the C-2 catalyst (figure 3(b)).

On the other hand, $H_2O$-O1 water molecule is also hydrogen bond to the O3 of a $PO_4^{3-}$ group, with a distance of 1.78 Å. In this case, determining which hydrogen bond is substituted by the OH$^-$ is harder, as they are energetically very similar. However, hypothesizing a combination of OH$^-$ ions pointing in both directions (O3$\cdots$H2 and H3$\cdots$O3) might be reasonable since the TSP treatment imposes a specific OH$^-$ orientation, whereas OH1$\cdots$H4 and O3$\cdots$H2 are pointing backwards.

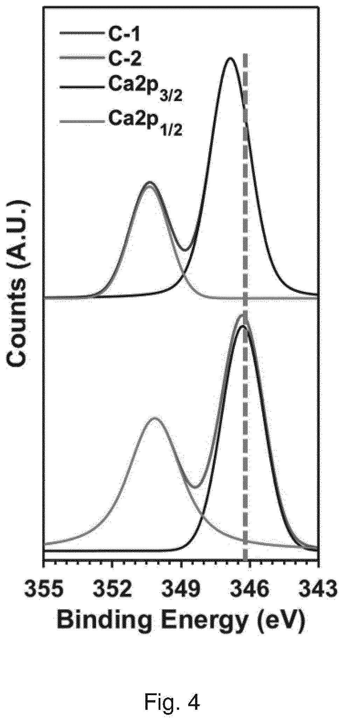

In order to quantify the enhancement in the lattice mobility of $Ca^{2+}$ ions highlighted by Raman spectroscopy, XPS measurements were conducted to capture the surface electron binding states of Ca. Figure 4 compares the XPS Ca2p of C-1 and C-2 catalysts. Both spectra present the characteristic $Ca\,2p_{3/2}$ and $Ca\,2p_{1/2}$ peaks of HAp located at 346.9 and 350.5 eV for C-1 and 346.4 and 350.0 eV for C-2. This observation is in good agreement with recent XPS studies on HAp after TSP treatment. The binding energy of the Ca 2p peak is mainly related with the Ca$\cdots PO_4^{3-}$ bonds. The standard value of Ca $2p_{3/2}$ is usually measured at -347.2 eV, even though shifts at higher binding energies are observed in some cases, for example when RCOO$^-$ groups are adsorbed (*i.e.* Ca$\cdots$COO$^-$ bonds are stronger than Ca$\cdots PO_4^{3-}$ bonds). Instead, shifts to smaller binding energies are appreciated for C-1 and C-2, which has been associated to the OH$^-$ vacancies generated by the TSP treatment. In agreement with the Raman spectra, the shift is more pronounced for the C-2 sample (-0.8 eV) than for the C-1 sample (-0.3 eV). This 0.5 eV difference has been attributed to the presence of the brushite co-existent phase in the C-2 catalyst.

## 8. Preparation of the cHAp/tsp-based catalyst

The 3-component catalyst was prepared by dropping successively 100 μL of 50 mM ATMP, 10 mM ZC and 50 mM ATMP aqueous solutions on a cHAp/tsp disk (diameter: 10 mm; thickness: 1 mm). Before each dropping step, the sample was kept 8 h at room temperature for drying.

## 9. Synthesis of amino acids

A high pressure stainless steel reactor was employed to perform the synthesis of amino acids. The reactor was also characterized by an inert reaction chamber coated with a perfluorinated polymer (120 mL) where both the catalyst and water were incorporated. The reactor was equipped with an inlet valve for the entrance of $N_2$, $CH_4$, $CO_2$ and an outlet valve to recover the gaseous reaction products. A UV lamp (GPH265T5L/4, 253.7 nm) was also placed in the middle of the reactor to irradiate the catalyst directly, the lamp being protected by a UV transparent quartz tube. All surfaces were coated with a thin film of a perfluorinated polymer in order to avoid any contact between the reaction medium and the reactor surfaces, in this way discarding other catalyst effects.

The reactions were performed at 95 °C for a reaction time of 48 h. Catalyst samples weighed approximately 150 mg and 0.5 mL of de-ionized liquid water were initially incorporated into the reaction chamber. The chamber was extensively purged with the first selected gas in order to eliminate the initial air content. Each selected gas was introduced to increase the reaction chamber pressure (measured at room temperature) to the target pressure. In all cases the chamber pressure was increased up to 6 bar by introducing sequentially 2 bar of each feeding reaction gas.

The reaction products were analyzed by NMR spectroscopy. All NMR spectra were acquired with a Bruker Avance III-400 spectrometer operating at frequencies of 400.1 and 100.6 MHz for $^1H$ and $^{13}C$, respectively. Chemical shifts were calibrated using tetramethylsilane ($^1H$ and $^{13}C$) as internal standard. Sixty-four and one thousand scans were recorded for $^1H$ and $^{13}C$ NMR, respectively. In order to remove the amino acids from the catalyst, samples were dissolved in deuterated water containing 100 mM of HCl and 50 mM of NaCl with the final addition of deuterated water.

10. cHAp: Temperature for the hydrothermal (HT) treatment

The precipitation and HT of HAp are crucial steps to adjust the stoichiometry and avoid the formation of other phases, such as β-tricalcium phosphate (βTCP) that can be easily formed depending on the conditions, and the sintering at 1000-1200 °C is frequently used to refine the crystal structure.

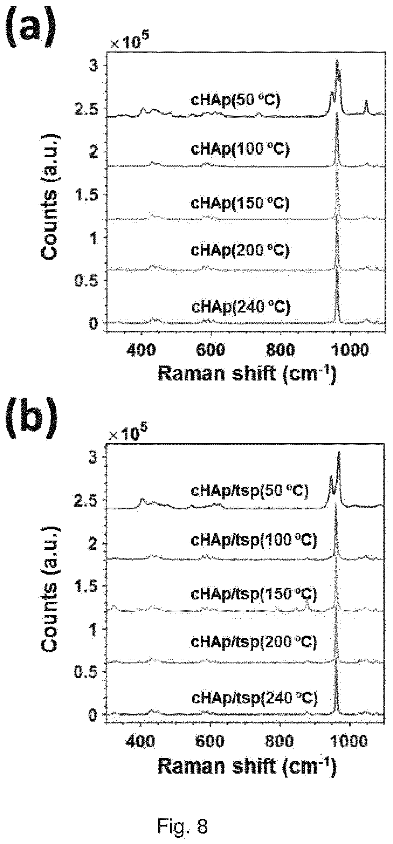

Figure 8 (a) compares the Raman spectra recorded for cHAp($T_h$) samples prepared by applying, after the precipitation step, a HT at $T_h$= 50 °C, 100 °C, 150 °C, 200 °C and 240 °C for 24 h. The values of the normal-mode frequencies of the $PO_4^{3-}$ tetrahedron typically observed from Raman measurements in aqueous solution are $\upsilon_1$= 938 cm$^{-1}$, $\upsilon_2$= 420 cm$^{-1}$, $\upsilon_3$= 1017 cm$^{-1}$ and $\upsilon_4$= 567 cm$^{-1}$. In cHAp and βTCP, the crystalline field causes not only the shifts but also the splitting of the $PO_4^{3-}$ normal modes, even though these effects depend on the crystallographic structure. The spectra displayed in figure 8 (a) clearly indicates that the splitting of the $PO_4^{3-}$ normal modes decreases with increasing hydrothermal temperature. For example, a single intense peak arising from the non-degenerate $\upsilon_1$, mode of $PO_4^{3-}$ at 962 cm$^{-1}$ is detected in the spectra of cHAp($T_h \geq$ 100 °C) samples, whereas several peaks can be observed for cHAp(50 °C). A similar feature is observed for the doubly degenerate $\upsilon_2$ and triply degenerate $\upsilon_3$ and $\upsilon_4$, which span a frequency range of 400-490, 570-625 and 1020-1095, respectively, for cHAp($T_h \geq$ 100 °C). In all cases the $PO_4^{3-}$ bands appear at frequencies 20-25 cm$^{-1}$ higher than those corresponding to the free-tetrahedral normal modes (*i.e.* in aqueous solution).

cHAp exhibits a P6$_3$/m space group and the unit cell contains six equivalent $PO_4^{3-}$ tetrahedrons, whereas βTCP crystallizes in the R3c space group and its unit cell contains 42 $PO_4^{3-}$ tetrahedrons distributed in three non-equivalent types. Consequently, the single intense peak

detected at $\upsilon_1$= 962 cm$^{-1}$ is observed as two peaks and a shoulder for βTCP. This structural difference also affects $\upsilon_2$ and $\upsilon_4$, which span over a higher frequency range in βTCP than in cHAp. Moreover, $\upsilon_2$ and $\upsilon_4$ are separated by a frequency gap of only 55 cm$^{-1}$ in βTCP, while the gap is of 120 cm$^{-1}$ in cHAp. As it can be seen in Figure 8 (a), the fingerprints of the spectrum recorded for the cHAp(50 °C) sample agree with those expected for βTCP, indicating that this is the predominant phase when the HT is performed at low temperature.

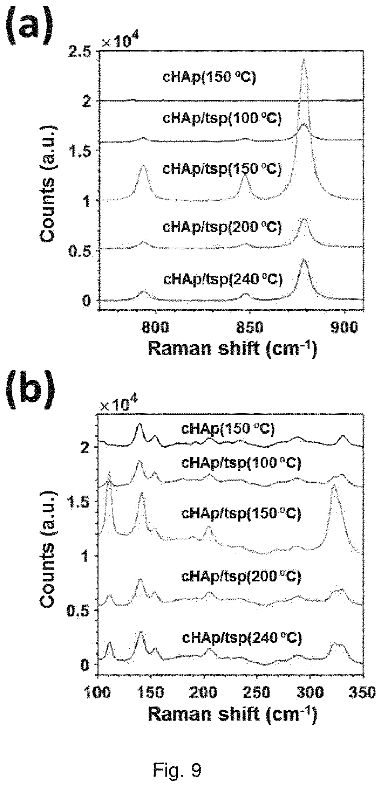

The Raman spectra obtained for cHAp/tsp($T_h$), which are displayed in figure 8(b), show similar features to those described for cHAp($T_h$) for the normal modes of $PO_4^{3-}$ (figure 8 (a)). After the TSP treatment, the intensity and width of the peaks associated to the $PO_4^{3-}$ correspond to the βTCP phase for the samples prepared at $T_h$= 50 °C, while cHAp is clearly identified in samples hydrothermally treated at $T_h \geq$ 100 °C. However, some distinctive features can be also identified in cHAp/tsp($T_h$) samples as a function of $T_h$. For example, cHAp/tsp(150 °C) shows weak peaks at around 330 cm$^{-1}$, whereas no signal is detected for the rest of the samples. Considering that the detection of these peaks depend on the degree of crystallinity of the sample, Raman results suggest that the highest crystallinity is achieved when the HT is performed at $T_h$= 150 °C. Furthermore, a peak at 878 cm$^{-1}$ is detected for cHAp/tsp($T_h \geq$ 100 °C), as is clearly in the evidenced in figure 9 (a). This signal, which is characteristic of the Brushite mineral (*i.e.* CaHPO$_4$·2H$_2$O) that is understood to be a precursor of apatite, has been attributed to the normal mode frequency of $HPO_4^{2-}$. The intensity of this band is much greater for the sample obtained at $T_h$= 150 °C, while for the rest it is observed as a weak peak. In addition, two bands typical of POH rotation and deformation modes appear at 794 and 848 cm$^{-1}$. Although they are very weak in all cases, POH signals also showed the highest intensity for samples obtained at $T_h$= 150 °C.

Figure 9 (b) compares the weak intensity bands observed between 100 and 350 cm$^{-1}$ for cHAp(150 °C) and cHAp/tsp($T_h \geq$ 100 °C). These bands have been attributed to translational modes of the Ca$^{2+}$ (111, 139 and 154 cm$^{-1}$), $PO_4^{3-}$ (287 cm$^{-1}$) and OH$^-$ (331 and 323 cm$^{-1}$) sublattices, and rotational modes of the $PO_4^{3-}$ group (205 cm$^{-1}$). As it can be seen, both the intensity and narrowness of all these bands is maximum when the TSP process is applied to cHAp(150 °C) samples. Moreover, the intensity bands associated to the translational modes of the OH$^-$ sublattice yield (331 and 323 cm$^{-1}$ in figure 9(b)) increases linearly with that of the POH rotation and deformation modes (794 and 848 cm$^{-1}$ in figure 9(a)), evidencing a structural correlation.

11. Duration of the HT and characteristics of the TSP treatment

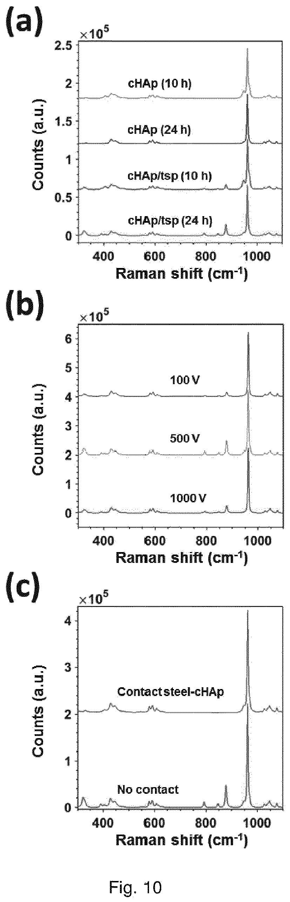

The duration of the HT is another factor that affects the structure of the HAp and, therefore, the performance of cHAp/tsp as catalyst. Figure 10 (a) compares the Raman spectra recorded for samples prepared at $T_h$= 150 °C when the time for the HT was of 10 or 24 h. As it can be seen, the spectra of the cHAp samples obtained after a HT of only 10 h show the peaks described in the previous section for the $\beta$TCP phase. Thus, the conversion of the $\beta$TCP phase into cHAp is only completed when the HT is long enough, even when the $T_h$ was the optimum. Interestingly, figure 10(a) shows that the TSP treatment favors the re-arrangement of the surface in samples prepared using both 10 and 24 h, as is proved by the apparition of the Brushite peak at 878 cm$^{-1}$ and the translational modes of the OH$^-$ sublattice at 323 cm$^{-1}$. Nevertheless, the $\beta$TCP fingerprints identified for cHAp(150 °C) samples obtained using a treatment time of 10 h, which are associated to the four normal-mode frequencies of the PO$_4^{3-}$, remain practically unaltered after the permanent polarization process. Accordingly, the TSP process cannot be used to compensate the undesirable structural effects induced by the shortening of the HT.

The effect of the electric field strength used to induce permanent polarization in cHAp has been examined by applying DC voltages of 100 V, 500 V or 1000 V (25, 125, 250 V·cm$^{-1}$ respectively) to cHAp(150 °C) samples. Although the Raman spectra obtained for all the resulting samples correspond to cHAp (figure 10(b)), some bands apparently associated to the catalytic activity of cHAp/tsp are slightly influenced by the strength of the DC voltage. More specifically, the peaks attributed to translation modes of the OH$^-$ sublattice (323 cm$^{-1}$), the normal mode of HPO$_4^{2-}$ (878 cm$^{-1}$), and the POH rotation and deformation modes (794 and 848 cm$^{-1}$) are more intense and better defined for samples polarized at 500 V than for those obtained at 100 V and 1000 V. According to these results, the alteration of the voltage used for the TSP process is not expected to annihilate the activity of cHAp/tsp as catalyst but to induce small changes in its effectivity.

Finally, the influence of the geometry of the electrodes in the TSP treatment has been investigated. For this purpose, cHAp/tsp(150 °C) samples were prepared using a DC voltage of 500 V and two different geometries for the electrodes: i) steel plates separated at 4 cm and, therefore, the cHAp(150 °C) disc was in contact with one electrode only (i.e. the thickness of the sintered mineral discs was 1 mm); and ii) steel plates separated at 1 mm and, therefore, each side of the cHAp(150 °C) disc was in contact with an electrode. The recorded Raman spectra, which are compared in figure 10(c), reveals that the bands at 794, 848 and 878 cm$^{-1}$ are only observed when the TSP process is conducted hindering the steel-cHAp contact. Also, the intensity of the band associated to the translational mode of the OH$^-$ sublattice (323 cm$^{-1}$) is much higher when the separation between the electrodes is of 4 cm.

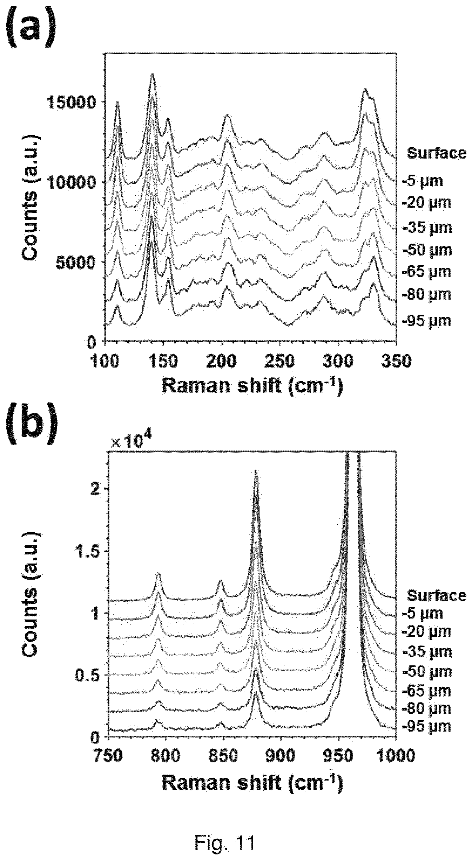

A depth profiling Raman analysis was conducted to monitor the extent of the changes induced by the TSP treatment. Figure 11(a) compares the Raman spectra recorded at different depths (i.e. from the surface to a depth of 95 $\mu$m) for cHAp/tsp(150 °C) prepared applying the HT for 24 h and using a polarizing voltage of 500 V at 1000 °C. Although the spectra show the same fingerprints in all cases, the intensity of the signals decreases with increasing depth. This observation is particularly remarkable for the bands of HPO$_4^{2-}$ (878 cm$^{-1}$) and both POH rotation and deformation modes (794 and 848 cm$^{-1}$). These results indicate that the changes caused by the TSP treatment are predominantly located at the surface of the mineral. This is corroborated in figure 11(b), which displays the intrinsically weak bands detected in the 110 and 330 cm$^{-1}$ region. The intensity of the bands associated to the rotational modes of the PO$_4^{3-}$ group (205 cm$^{-1}$) and, specially, to the translational modes of the Ca$^{2+}$ (111, 139 and 154) cm$^{-1}$), PO$_4^{3-}$ (287 cm$^{-1}$) and OH$^-$ (323 cm$^{-1}$) sublattices decreases with increasing depth.

## 12. Morphological and structural characterization of cHAp samples

Raman spectra displayed in figures 8 and 9 indicate that the chemical properties of cHAp($T_h$) and cHAp/tsp($T_h$) depends on $T_h$ and, therefore, the catalytic activity of the latter. To investigate the implications of $T_h$ in the morphology and structure of cHAp, SEM and XRD have been used.

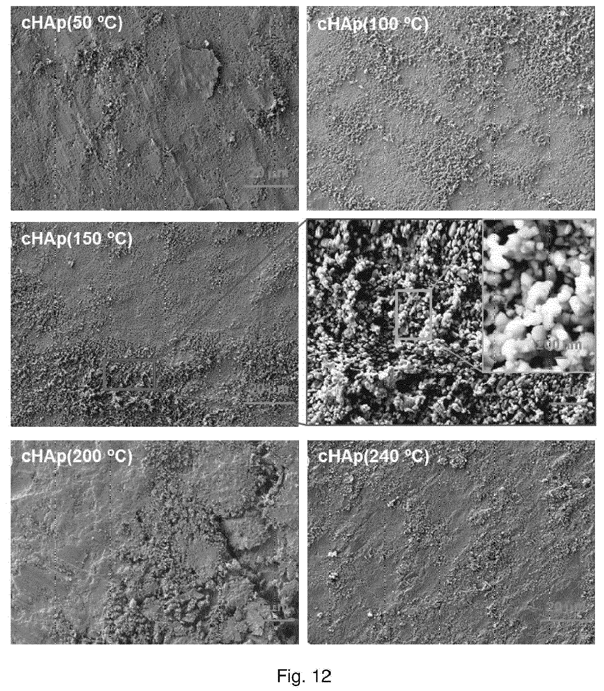

Low magnification SEM micrographs of cHAp($T_h$) with $T_h$= 50 °C, 100 °C, 150 °C, 200 °C and 240 °C are displayed in figure 12. With exception of the sample with $\beta$TCP ($T_h$= 50 °C), which presents the most compact surface, the rest of the samples presents a morphology made of compact regions alternated with extensive porous zones. Inspection of the high magnification micrographs, shown in figure 12 for cHAp(150 °C) as representative sample, indicate that porous zones are constituted by pillars that grow through the aggregation of mineral nanoparticles in a preferential direction. However, the morphology of the porous areas is apparently independent of $T_h$.

Structural characterization of prepared cHAp($T_h$) samples was completed by WAXD (Figure 13(a)). The characteristic fingerprint of hexagonal crystal symmetry cHAp ($a$= $b$= 9.421 Å, $c$= 6.881 Å, $\alpha$= $\beta$= 90°, and $\gamma$= 120°; JCPDS card number 9-0432) is typically associated to the peaks at 32°-34° $2\theta$, which correspond to the (211), (112) and (300) reflections. These reflections are clearly identified in the diffraction profiles of all samples prepared using $T_h \geq$ 100 °C and can be intuited in that of the cHAp(50 °C) sample. Other characteristic reflection peaks of cHAp appear at $2\theta$= 32°, 34°, 40°, 47° and 49°, which correspond to (211), (202) (130) (222) and (214) reflections. The (112) and (300) peaks were also used to determine the crystallinity ($\chi_c$: Eqn 1), whereas the (211) reflection was used to calculate the crystallite size ($L_{211}$; Eqn 2). The $\chi_c$ of cHAp($T_h$) is 0.53, 0.82, 0.68 and 0.77 for $T_h$= 100 °C, 150 °C, 200 °C and 240 °C, respectively, which is fully consistent with Raman spectra displayed in figure 8(a). Thus, the $\chi_c$ of cHAp is maximum when it is prepared at $T_h$= 150 °C followed by that obtained at $T_h$= 240 °C. The variation detected in cHAp(200 °C) with a decrease in $\chi_c$ in relation to cHAp(150 °C) and cHAp(240 °C), and which is experimentally reproducible, has been attributed to combined effect of the lyophilization and sintering processes on the crystals, which seems to depend on the value of $T_h$ used. This could not only explain the reduction of $\chi_c$ when $T_h$ increases from 150 °C to 200 °C but also the fact that $\chi_c$ is lower for cHAp(240 °C) than for cHAp(150 °C). Besides, the calculated $L_{211}$ values correspond to 69.1, 82.7, 82.6 and 82.7 nm for cHAp($T_h$) with $T_h$= 100 °C, 150 °C, 200 °C and 240 °C, respectively.

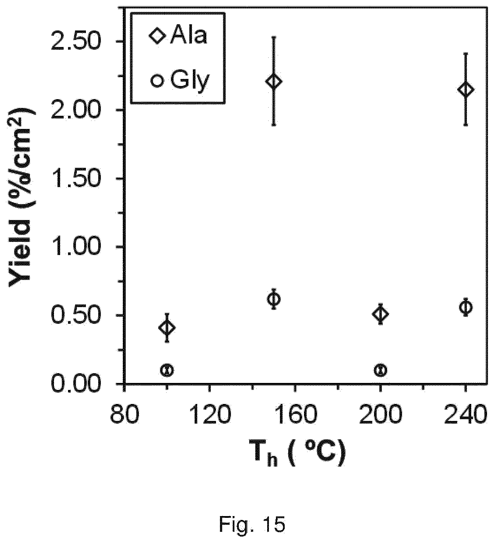

On the other hand, the most relevant reflection peaks of brushite (JCPDS card number 72-0713), which has a monoclinic structure with cell parameters $a$= 5.812 Å, $b$= 15.180 Å, $c$= 6.239 Å, $\alpha=\gamma$= 90° and $\beta$= 116.43°, are clearly observed in the X-ray diffraction pattern of cHAp(150 °C) (figure 15a). These reflections, which are weaker for cHAp(240 °C) and much weaker for cHAp(100 °C) and cHAp(200 °C), correspond to the $(141)$, $(121)$, $(15\bar{2})$ and $(14\bar{3})$ with $2\theta$= 29°, 35°, 42° and 51°, respectively. It is worth mention that, although the position of some of these reflections, as for example the $(14\bar{3})$, matches other reflections found in the theoretical diffraction of pure cHAp, the changes in the relative intensities points to co-existence of the two phases, which is in agreement with Raman spectra. The amounts of cHAp and brushite phases in samples prepared at $T_h\geq$ 100 were roughly estimated using X-ray diffraction patterns by comparing the $(211)$ reflection of cHAp and the $(141)$ reflection of brushite. Results show that, although the formation of the latter phase is promoted when the HT is conducted at $T_h\geq$ 100 °C (figure 13(b)), cHAp is clearly the predominant phase in all cases. However, the content of Brushite increases from ~5% for $T_h$= 100 °C and 200 °C to ~15% for $T_h$= 150 and 240 °C.

In general the anomalous behaviour of the samples obtained at $T_h$= 200 °C has been attributed to the dehydration process reported for Brushite at such temperature. Thus, the layered structure of Brushite, $CaHPO_4 \cdot 2H_2O$, in which mineral layers are held together by hydrogen bonded water molecules, converts into an amorphous phase and Monetite, $CaHPO_4$. More specifically, although surface water evaporates at around 100 °C, the two crystallographic water molecules of Brushite, which are associated by hydrogen bonds with oxygen atoms in phosphate group, remain stable at such temperature, leaving from the system at 200 °C. The structural transitions associated to the Brushite dehydration at 200 °C explains the reduction of the $\chi_c$, which in turn is in detriment of the catalytic activity.

Finally, inspection of the diffractogram obtained for cHAp(50 °C) (figure 13(a)) allows to recognize the reflection peaks typically reported for βTCP (JCPDS card number 09016), which predominate over those associated to the cHAp. Thus, the rhombohedral βTCP appears as the predominant crystalline phase in samples HT-treated at the lowest temperature.

## 13. Performance of cHAp/tsp($T_h$) as catalyst for the synthesis of amino acids

In a recent study, the inventors catalyzed the fixation of nitrogen from $N_2$ and carbon from $CO_2$ and $CH_4$ to obtain Gly (glycine) and Ala (alanine), the two simplest amino acids. The catalyst was prepared by coating cHAp/tsp(150 °C) samples with two layers of ATMP separated by an intermediate ZC layer. For this purpose, the cHAp/tsp(150 °C) disks were sequentially immersed in 5 mM ATMP, 5 mM ZC and 1.25 mM ATMP aqueous solutions at room temperature for 5 h. After each immersion, samples were dried at 37 °C for 3 h. The catalyzed reaction was conducted under UV light irradiation and mild reaction conditions, in an inert reaction chamber starting from a simple gas mixture containing $N_2$, $CO_2$, $CH_4$ and $H_2O$.

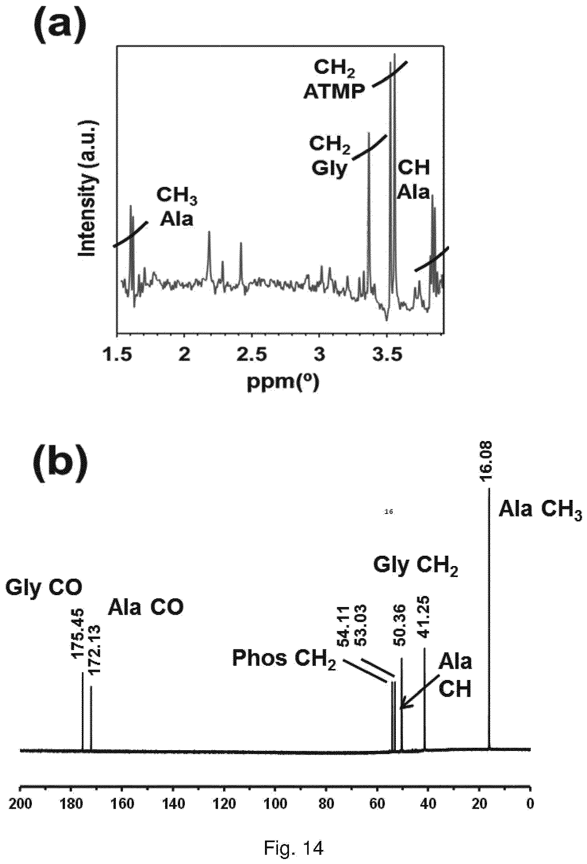

Figure 14 proves that change in the application of the ATMP and ZC coatings does not alter the performance of the catalyst. Figure 14(a) shows the $^1$H NMR spectrum of samples obtained by dissolving the catalyst prepared using cHAp(150 °C) and the products of reaction after 48 h at 95 °C. The signal corresponding to ATMP methylene group appears as doublet at 3.53-3.56 ppm, while the signals corresponding to the methylene group of produced Gly is singlet at 3.37 ppm and both methine and methyl groups of Ala are the quadruplet at 3.84-3.87 ppm and the doublet at 1.60-1.62 ppm, respectively. The same compounds are also detected in the $^{13}$C NMR spectrum displayed in figure 14(b), where only peaks assigned to the ATMP (53.82 and 52.92 ppm), Gly (172.26 and 41.35 ppm) and Ala (175.26, 50.56 and 16.18 ppm) units are detected.

It should be noted that the reaction was positive also for catalysts prepared using cHAp($T_h$) with $T_h\geq$ 100°C. The yield of the reaction was calculated using commercial Gly and Ala (purchased from Sigma-Aldrich) at a controlled concentration to calibrate the $^1$H NMR peaks. The variation of the yield of the reaction expressed in % per $cm^2$ of catalyst against $T_h$ is represented in Figure 15. These values are higher than those found in the inventor's previous study, which was 2.5 after 48 h (i.e. Gly/Ala ratio decreased from 5.4 to 2.2 when the reaction time increased from 2 to 96h). This feature has been attributed to the presence of the brushite phase, which was not observed in the catalyst prepared in the inventor's previous study. Accordingly, the combination of brushite and cHAp achieved with the process described in this work apparently accelerates the transformation of Gly into Ala.

On the other hand, the yield correlates with both the $\chi_c$ and the brushite content in the cHAp/tsp($T_h$). Thus,

the maximum yield of amino acids after 48 h was obtained for the catalysts prepared at $T_h$= 150 °C and 240 °C (i.e. 2.8% and 2.7%, respectively), which displayed not only the highest $\chi_c$ (*i.e.* 0.82 and 0.77, respectively) but also the highest content of brushite (*i.e.* 15% and 12%, respectively). In contrast, the total yield decreases one order of magnitude, ~0.5-0.6%, for reactions catalyzed by cHAp/tsp($T_h$) samples with $\chi_c$ < 0.7. This feature clearly reflects the very important role of the spatially translations modes, which facilitates the transport of charge at the surface through oscillation and translational molecular movements upon excitation of the lattice.

**Claims**

1. A process for producing amino acids, in particular glycine and/or alanine, comprising the step of

   - contacting a gas mixture comprising or consisting of carbon dioxide, methane and nitrogen in presence of water with a catalyst comprising or consisting of permanently polarized hydroxyapatite and brushite and/or a brushite-like material.

2. The process according to claim 1, **characterized in that** the catalyst is in the form of a multi-phase catalyst, wherein the permanently polarized hydroxyapatite forms a phase, in particular a main phase, of the catalyst and the brushite and/or brushite-like material forms a further phase of the catalyst.

3. The process according to claim 1 or 2, **characterized in that** the catalyst has a wide angle x-ray scattering pattern as shown on figure 1(a).

4. The process according to any of the preceding claims, **characterized in that** the catalyst has a Raman spectrum as shown on figure 1(b).

5. The process according to any of the preceding claims, **characterized in that** the permanently polarized hydroxyapatite has a proportion which is larger than a proportion of the brushite and/or the brushite-like material.

6. The process according to any of the preceding claims, **characterized in that** the permanently polarized hydroxyapatite has a proportion of 50 % by weight to 99.9 % by weight, in particular 85 % by weight to 90 % by weight, preferably 85 % by weight to 88 % by weight, based on the total weight of the catalyst.

7. The process according to any of the preceding claims, **characterized in that** the brushite and/or the brushite-like material has a proportion of > 0 % by weight to 50 % by weight, in particular 10 % by weight to 15 % by weight, preferably 12 % by weight to 15 % by weight, based on the total weight of the catalyst.

8. The process according to any of the preceding claims, **characterized in that** the contacting step is carried out in the presence of liquid water and/or water vapor.

9. The process according to any of the preceding claims, **characterized in that** the contacting step is carried out with a volumetric ratio of the catalyst to the water, in particular liquid water and/or water vapor, of 1000 : 1 to 0.01 : 1, in particular 500 : 1 to 0.05 : 1, preferably 300 : 1 to 350 : 0.01.

10. The process according to any of the preceding claims, **characterized in that** the contacting step is carried out with a volumetric ratio of the carbon dioxide to the methane to the nitrogen of 100 : 1 : 1 or 1 : 100 : 1 or 1 : 1 : 100, in particular 10 : 1 : 1 or 1 : 10 : 1 or 1 : 1 : 10, preferably 1 : 1 : 1.

11. The process according to any of the preceding claims, **characterized in that** the contacting step is carried out under a total pressure of > 0 bar to 250 bar, in particular > 0 bar to 100 bar, preferably > 0 bar to 10 bar.

12. The process according to any of the preceding claims, **characterized in that** the contacting step is carried out under a partial pressure of the carbon dioxide of > 0 bar to 100 bar, in particular > 0 bar to 50 bar, preferably > 0 bar to 5 bar and/or under a partial pressure of the methane of > 0 bar to 100 bar, in particular > 0 bar to 50 bar, preferably > 0 bar to 10 bar and/or under a partial pressure of the nitrogen of > 0 bar to 100 bar, in particular > 0 bar to > 50 bar, preferably > 0 bar to 10 bar.

13. The process according to any of the preceding claims, **characterized in that** the contacting step is carried out with a molar ratio of the carbon dioxide to the catalyst of > 0 to 0.5, in particular 0.2 to 0.5, preferably 0.3 to 0.5, and/or with a molar ratio of the methane to the catalyst of > 0 to 0.5, in particular 0.2 to 0.5, preferably 0.3 to 0.5 and/or with a molar ratio of the nitrogen to the catalyst of > 0 to 0.5, in particular 0.2 to 0.5, preferably 0.3 to 0.5.

14. The process according to any of the preceding claims, **characterized in that** the contacting step is carried out under UV irradiation or UV-Vis irradiation, in particular having a wavelength from 200 nm to 850 nm, in particular 240 nm to 400 nm, preferably 250 nm to 260 nm, more preferably of 253.7 nm, and/or having a irradiance from 0,1 W/m² to 200 W/m², in

particular 1 W/m$^2$ to 50 W/m$^2$, preferably 2 W/m$^2$ to 10 W/m$^2$.

15. The process according to any of the preceding claims, **characterized in that** the contacting step is carried out at a temperature of 25 °C to 250 °C, in particular 95 °C to 140 °C, preferably of 95 °C.

16. Use of a process according to any of the preceding claims for removing harmful gases such as carbon dioxide, carbon monoxide, methane or mixtures thereof from an atmosphere, in particular from air.

Fig. 1 (a)

Fig. 1 (b)

Fig. 1 (c)

Fig. 1 (d)

Fig. 2

Fig. 3

Fig. 4

Fig. 5(a)

Fig. 5(b)

Fig. 6(a)

Fig. 6(b)

Fig. 7(a)

Fig. 7(b)

Fig. 8

Fig. 9

Fig. 10

Fig. 11

Fig. 12

Fig. 13

Fig. 14

Fig. 15

Fig. 16

Europäisches
Patentamt

European
Patent Office

Office européen
des brevets

# EUROPEAN SEARCH REPORT

Application Number

EP 20 38 3002

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 278 818 A1 (B BRAUN SURGICAL S A [ES]; UNIV POLITÈCNICA DE CATALUNYA [ES]) 7 February 2018 (2018-02-07) * tables 1-4 * * paragraphs [0035] - [0038] * ----- | 1-16 | INV. A61L15/00 B01J27/18 C01B25/32 B01D53/00 C07C227/00 |
| T | SANS JORDI ET AL: "Optimization of Permanently Polarized Hydroxyapatite Catalyst. Implications for the Electrophotosynthesis of Amino Acids by Nitrogen and Carbon Fixation", JOURNAL OF CATALYSIS, 1 March 2021 (2021-03-01), XP055792888, US ISSN: 0021-9517, DOI: 10.1016/j.jcat.2021.03.023 Retrieved from the Internet: URL:https://www.sciencedirect.com/science/article/pii/S0021951721001275/pdfft?md5=7e416861005acff2f541bb26ea2dd43b&pid=1-s2.0-S0021951721001275-main.pdf> * paragraphs [02.2], [0004] * ----- | | |

TECHNICAL FIELDS SEARCHED (IPC)

A61L
B01J
C01B
C07C
B01D

The present search report has been drawn up for all claims

| Place of search | Date of completion of the search | Examiner |
|---|---|---|
| The Hague | 7 April 2021 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 3002

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2021

| Patent document cited in search report | | Publication date | Patent family member(s) | Publication date |
|---|---|---|---|---|
| EP 3278818 | A1 | 07-02-2018 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018024727 A1

**Non-patent literature cited in the description**

- **ITOH, S. ; NAKAMURA, S. ; KOBAYASHI, T. ; SHINOMIYA, K. ; YAMASHITA, K. ; ITOH, S.** Effect of Electrical Polarization of Hydroxyapatite Ceramics on New Bone Formation. *Calcif. Tissue Int.,* 2006, vol. 78, 133-142
- **NAKAMURA, S. ; KOBAYASHI, T. ; YAMASHITA, K.** Highly Orientated Calcification in Newly Formed Bones on Negatively Charged Hydroxyapatite Electrets. *Key Eng. Mater.,* 2005, 284-286, 897-900

- **RIVAS, M. ; DEL VALLE, L. J ; ARMELIN, E ; BERTRAN, O. ; TURON, P. ; PUIGGALÍ, J. ; ALEMÁN, C.** Hydroxyapatite with Permanent Electrical Polarization: Preparation, Characterization, and Response against Inorganic Adsorbates. *Chem. Phys. Chem.,* 2018, vol. 19, 1746-1755
- **RIVAS, M ; DEL VALLE, L. J. ; TURON, P. ; ALEMÁN, C. ; PUIGGALÍ, J.** Sustainable Synthesis of Amino Acids by Catalytic Fixation of Molecular Dinitrogen and Carbon Dioxide. *Green Chem.,* 2018, vol. 20, 685-693